# EUROPEAN PATENT SPECIFICATION

(11) **EP 3 291 821 B1**
(45) Date of publication and mention of the grant of the patent: **25.02.2026**
(21) Application number: 16790145.3
(22) Date of filing: 06.05.2016
(51) Int. Cl.: A61K 35/15, A61K 39/39, A61K 38/17, A61K 38/21, A61K 31/4745, A61K 31/708, A61K 31/713, A61K 40/10, A61K 40/19, A61K 40/24, A61K 40/42, A61K 45/06, C12N 5/0784, A61K 39/00, A61P 35/00, A61P 37/04

(54) **DENDRITIC CELL IMMUNOTHERAPY**
IMMUNTHERAPIE MIT DENDRITISCHEN ZELLEN
IMMUNOTHÉRAPIE PAR CELLULES DENDRITIQUES

(30) Priority: 07.05.2015 US 201562158237 P
(43) Date of publication of application: 14.03.2018
(73) Proprietor: Baylor College of Medicine, Houston, TX 77030 (US)
(72) Inventor: DECKER, William, K., Houston, TX 77030 (US); HALPERT, Matthew, Houston, TX 77030 (US)
(74) Representative: Vossius & Partner Patentanwälte Rechtsanwälte mbB
(86) International application number: PCT/US2016/031157
(87) International publication number: WO 2016/179475

(56) References cited:
- WO-A1-2009/155322
- WO-A1-2012/140130
- WO-A1-94/02156
- WO-A2-03/020884
- WO-A2-2005/123112
- WO-A2-2012/140127
- US-A1- 2003 138 413
- US-A1- 2006 057 129
- US-A1- 2012 328 662
- US-A1- 2015 010 613
- VICTORIA HOENE ET AL: "Human monocyte-derived dendritic cells express TLR9 and react directly to the CpG-A oligonucleotide D19", JOURNAL OF LEUKOCYTE BIOLOGY, vol. 80, no. 6, 25 September 2006 (2006-09-25), US, pages 1328 - 1336, XP055441637, ISSN: 0741-5400, DOI: 10.1189/jlb.0106011
- W CHEN: "Enhancement of antigen-presenting ability of B lymphoma cells by immunostimulatory CpG-oligonucleotides and anti-CD40 antibody", IMMUNOLOGY LETTERS, vol. 77, no. 1, 1 May 2001 (2001-05-01), pages 17 - 23, XP055209683, ISSN: 0165-2478, DOI: 10.1016/S0165-2478(01)00189-4
- XIA YUMIN ET AL: "CpG oligodeoxynucleotide as immune adjuvant enhances photodynamic therapy response in murine metastatic breast cancer", JOURNAL OF BIOPHOTONICS, WILEY - VCH VERLAG, DE, vol. 7, no. 11-12, 31 October 2014 (2014-10-31), pages 897 - 905, XP009508682, ISSN: 1864-063X, [retrieved on 20130807], DOI: 10.1002/JBIO.201300072
- NAKAHARA NORIMOTO ET AL: "Effective induction of antiglioma cytotoxic T cells by coadministration of interferon-[beta] gene vector and dendritic cells", CANCER GENE THERAPY, NATURE PUBLISHING GROUP US, NEW YORK, vol. 10, no. 7, 30 June 2003 (2003-06-30), pages 549 - 558, XP037756849, ISSN: 0929-1903, [retrieved on 20030630], DOI: 10.1038/SJ.CGT.7700598
- KIM ET AL.: "AIMP1/p43 protein induces the maturation of bone marrow-derived dendritic cells with T helper type 1-polarizing ability", J IMMUNOL., vol. 180, no. 5, 1 March 2008 (2008-03-01), pages 2894 - 2902, XP008136870
- YANO ET AL.: "Ipilimumab augments antitumor activity of bispecific antibody-armed T cells", J TRANSL MED., vol. 12, no. 191, 9 July 2014 (2014-07-09), pages 1 - 11, XP021194806
- RAMOS ET AL.: "RIG-I like receptors and their signaling crosstalk in the regulation of antiviral immunity", CURR OPIN VIROL., vol. 1, no. 3, 1 September 2011 (2011-09-01), pages 167 - 176, XP055329361

## Description

### BACKGROUND OF THE INVENTION

### 1. Field of the Invention

The present invention is generally in the field of molecular biology, immunology and medicine. More particularly, it concerns a primed dendritic cell population for use in the treatment of a cancer or tumor in a subject, as characterized in the claims.

### 2. Description of Related Art

As critical T_{H}1-associated effectors, CD8⁺ cytotoxic T-cells (CTLs) are attractive targets for therapeutic intervention as their effector functions are crucial to antiviral and anti-tumor immunity and, as such, to the survival of the host (Dudda *et al.,* 2013). T-cell activation requires interactions with professional antigen presenting cells (APC), of which dendritic cells (DC) are most highly specialized in antigen processing and presentation, lymphocyte co-stimulation, and the generation of cytokines and other inflammatory mediators that modulate terminal T-cell differentiation (Lotze *et al.,* 2001). DC are equipped to both promote T-cell polarization as well as to become T_{H}1 polarized themselves (*e.g*. DC1) (Hokey *et al.,* 2005). DC1-polarization may be fostered by various combinations of inflammatory cytokines (Hilkens *et al.,* 1997), interferons (Longhi *et al.,* 2009), and pattern-recognition receptor (PRR) agonists including toll-like receptor (TLR) ligands (Spranger *et al.,* 2010), but confounding data derived from TLR^{-/-}, MyD88^{-/-}, type I interferon (IFN)^{-/-}, and type I IFNR^{-/-} systems allude to additional, unidentified mechanisms regulating DC polarization (Ahmed *et al.,* 2009; Carvalho *et al.,* 2011; Lopez *et al.,* 2003; Lopez *et al.,* 2006A; Lopez *et al.,* 2006B; Tam and Wick, 2009). Furthermore, attempts to enhance T-cell help and DC licensing by use of immunogenic, heterologous class II peptides (Jones *et al.,* 1999; Rosa *et al.,* 2004) successfully enhanced CD40 signaling but paradoxically also downregulated antigen-specific CD8⁺ CTL in some models (Hung *et al.,* 2007; Kim *et al.,* 2008; Ressing *et al.,* 2000). Other studies indicate that influenza-specific T_{H}1 immunity can be generated normally in mice lacking CD4⁺ T-cells (Allan *et al.,* 1990) but is defective in mice lacking MHC Class II (Tripp *et al.,* 1995), indicating a potential role for MHC in T_{H} polarization. Despite significant mechanist investigation into the factors that modulate dendritic cell antigen presentation and function, until now it has remained unclear how dendritic cell antigen presentation might be modulated to enhance immune stimulation.

### SUMMARY OF THE INVENTION

The present invention relates to the embodiments as characterized in the claims. Accordingly, the present invention relates to a primed dendritic cell population for use in the treatment of a cancer or tumor in a subject, wherein the cells have been primed with at least one antigen associated with the cancer or at least one tumor antigen, and wherein the primed dendritic cell population is to be administered to the subject in conjunction with interferon-α (IFN-α).

Further disclosed for reference is a method for providing an immune response in a subject having a diseased cell population comprising obtaining a primed dendritic cell population, wherein the cells have been primed with at least one antigen specific to the diseased cell population and administering an effective amount the primed dendritic cell population to the subject. A primed dendritic cell population may be administered in conjunction with a Type I interferon (INF), a TLR-7 agonist, a TLR-9 agonist, AIMp1, a TLR-3 agonist, a retinoic acid inducible gene-1 (RIG-1)-like receptor ligand or a cytosolic DNA (CDS) receptor ligand. In further aspects, a primed dendritic cell population is administered to a lymphoid tissue site proximal to the diseased cell population in the subject. Furthermore, a primed dendritic cell population may be administered in conjunction with a Type I interferon (INF), a TLR-7 agonist, a TLR-9 agonist, AIMp1 TLR-3 agonist, a retinoic acid inducible gene-1 (RIG-1)-like receptor ligand or a cytosolic DNA (CDS) receptor ligand and may be administered to a lymphoid tissue site proximal to the diseased cell population in the subject.

Further disclosed is administration of a primed dendritic cell population in conjunction with a Type I interferon (INF), a TLR-7 agonist, a TLR-9 agonist, AIMp1 or a mixture thereof. For example, in some cases, the primed dendritic cell population is administered in conjunction with a Type I INF. The Type I INF is INF-α. Furthermore, the primed dendritic cell population may be administered in conjunction with a TLR-7 agonist. The TLR-7 agonist may be selected from the group consisting of CL075, CL097, CL264, CL307, GS-9620, Poly(dT), imiquimod, gardiquimod, resiquimod (R848), loxoribine, and a ssRNA oligonucleotide. Furthermore, the primed dendritic cell population may be administered in conjunction with a TLR-9 agonist. For example, in some cases, the TLR-9 agonist may be a CpG oligodeoxynucleotide (CpG ODN). Furthermore, the primed dendritic cell population may administered in conjunction with AIMp1 polypeptide (see, *e.g.,* NCBI accession numbers NP_001135887.1 and NP_001135888.1). Furthermore, the primed dendritic cell population my be administered in conjunction with a TLR-3 agonist. Particularly, the TLR-3 agonist may be polyinosine-polycytidylic acid (poly(I:C)) or RGC100. Furthermore, the primed dendritic cell population may be administered in conjunction with a RIG-1-like receptor ligand. The RIG-1-like receptor ligand may be further defined as a RIG-1, MDA5, LGP2, or IPS-1 ligand. For example, the RIG-1-like receptor ligand is selected from the group consisting of a MDA5 ligand, a LGP2 ligand, a ssRNA, a dsRNA, 5'ppp-dsRNA, Poly(dA:dT), and Poly(I:C). Furthermore, the primed dendritic cell population may be administered in conjunction with a CDS receptor ligand. The CDS receptor ligand may be further defined as a cGAS-STING ligand. For example, the cGAS-STING ligand is bacterial cyclic-dinucleotides (CDNs).

Furthermore, the Type I INF, TLR-7 agonist, TLR-9 agonist, AIMp1, TLR-3 agonist, a retinoic acid inducible gene-1 (RIG-1)-like receptor ligand or a cytosolic DNA (CDS) receptor ligand may administered before, after or essentially simultaneously with the primed dendritic cell population. Furthermore, the Type I INF, TLR-7 agonist, TLR-9 agonist or AIMp1 may be administered systemically and the dendritic cell population may be administered locally. Furthermore, the Type I INF, TLR-7 agonist, TLR-9 agonist or AIMp1 and the dendritic cell population may be both administered locally, such as to a site proximal to the diseased cell population in the subject. Particularly, the Type I INF, TLR-7 agonist, TLR-9 agonist or AIMp1 may be administered within about 1 week, 1 day, 8 hours, 4 hours, 2 hours or 1 hour of the primed dendritic cell population. Furthermore, it is possible that a subject being administered a primed dendritic cell population has been previously treated or is currently being treated with a Type I INF, TLR-7 agonist, TLR-9 agonist or AIMp1. Furthermore, the method may further comprise administering a composition comprising an effective amount of the primed dendritic cell population and a Type I INF, a TLR-7 agonist, a TLR-9 agonist or AIMp1 to the subject.

In still further aspects, an immune checkpoint inhibitor is to be administered to the subject (in conjunction with a primed dendritic cell composition). For example, in some aspects, the immune checkpoint inhibitor is a CTLA-4 antagonist. In some aspects, CTLA-4 antagonist is a small molecule inhibitor or an inhibitor nucleic acid specific to CTLA-4. In certain aspects, the inhibitory nucleic acid is a RNA. In further aspects, the RNA is a small interfering RNA (siRNA) or a short hairpin RNA (shRNA). In further aspects, a CTLA-4 antagonist is a CTLA-4-binding antibody. In some aspects, the antibody is a monoclonal antibody or a polyclonal antibody, In some aspects, a CTLA-4-binding antibody may be an IgG (*e.g.,* IgG1, IgG2, IgG3 or IgG4), IgM, IgA, genetically modified IgG isotype, or an antigen binding fragment thereof. The antibody may be a Fab', a F(ab')2 a F(ab')3, a monovalent scFv, a bivalent scFv, a bispecific or a single domain antibody. The antibody may be a human, humanized, or de-immunized antibody. In still further aspects, the immune checkpoint inhibitor is ipilimumab, pembrolizumab or nivolumab.

In certain aspects of the embodiments, a primed dendritic cell population is administered to a lymphoid tissue site proximal to the diseased cell population in the subject. Furthermore, the primed dendritic cell population may be administered in conjunction with a Type I INF, an agonist of TLR-7, an agonist of TLR-9, AIMp1, a TLR-3 agonist, a retinoic acid inducible gene-1 (RIG-1)-like receptor ligand or a cytosolic DNA (CDS) receptor ligand and the primed dendritic cell population may be administered to a lymphoid tissue site proximal to the diseased cell population in the subject. In specific aspects, said lymphoid tissue site is lymphoid tissue that drains tissue surrounding the diseased cell population. For example, a primed dendritic cell population is, in some aspects, administered to a lymph node that that drains tissue surrounding the diseased cell population. In some specific aspects, a primed dendritic cell population is administered to the subsegmental, segmental, lobar, interlobar, hilar, mediastinal, supratrochlear, deltoideopectoral, lateral, pectoral, subscapular, intermediate, subclavicular, superficial inguinal, deep inguinal, popliteal, facial buccinators, facial nasolabial, prostate, mandibular, submental, occipital, mastoid/retroauricular, parotid, deep preauricular, deep infra-auricular, deep intraglandular, deep cervical, deep anterior cervical, pretracheal, paratracheal, prelaryngeal, thyroid, deep lateral cervical, superior deep cervical, inferior deep cervical, retropharyngeal, jugulodigastric, anterior cervical, lateral cervical, supraclavicular, retroaortic, lateral aortic, celiac, gastric, hepatic, splenic, superior mesenteric, mesenteric, ileocolic, mesocolic, inferior mesenteric, or pararectal lymph nodes. In some aspects, a dendritic cell population is administered by direct injection into a lymph node.

A subject for treatment according to the disclosure may have a cancer, an autoimmune disease or an infectious disease. Examples of autoimmune diseases include, but are not limited to, Coeliac disease, diabetes mellitus type 1 (IDDM), systemic lupus erythematosus (SLE), Sjögren's syndrome, multiple sclerosis (MS), Hashimoto's thyroiditis, Graves' disease, idiopathic thrombocytopenic purpura, rheumatoid arthritis (RA), acute idiopathic thrombocytopenic purpura, chronic idiopathic thrombocytopenic purpura, dermatomyositis, Sydenham's chorea, myasthenia gravis, systemic lupus erythematosus, lupus nephritis, rheumatic fever, polyglandular syndromes, bullous pemphigoid, diabetes mellitus, Henoch-Schonlein purpura, post-streptococcalnephritis, erythema nodosurn, Takayasu's arteritis, Addison's disease, rheumatoid arthritis, multiple sclerosis, sarcoidosis, ulcerative colitis, erythema multiforme, IgA nephropathy, polyarteritis nodosa, ankylosing spondylitis, Goodpasture's syndrome, thromboangitisubiterans, Sjogren's syndrome, primary biliary cirrhosis, Hashimoto's thyroiditis, thyrotoxicosis, scleroderma, chronic active hepatitis, polymyositis/dermatomyositis, polychondritis, pamphigus vulgaris, Wegener's granulomatosis, membranous nephropathy, amyotrophic lateral sclerosis, tabes dorsalis, giant cell arteritis/polymyalgia, perniciousanemia, rapidly progressive glomerulonephritis, psoriasis, and fibrosing alveolitis. Examples of infectious diseases include, but are not limited to, anthrax, chickenpox, diphtheria, hepatitis A, B or C, HIB, HPV, HIV, Lyme disease, seasonal influenza, encephalitis, malaria, measles, meningitis, mumps, pertussis, polio, rabies, rubella, shingles, smallpox, tetanus, TB and yeller fever.

In some aspects of the embodiments, the diseased cell population treated by compositions of the embodiments are cancer cells. Cancer cells that may be treated according to the embodiments include but are not limited to cells from the bladder, blood, bone, bone marrow, brain, breast, colon, esophagus, gastrointestine, gum, head, kidney, liver, lung, nasopharynx, neck, ovary, prostate, skin, stomach, testis, tongue, or uterus. In some aspects, the cancer may be a neoplasm, malignant; carcinoma; carcinoma, undifferentiated; giant and spindle cell carcinoma; small cell carcinoma; papillary carcinoma; squamous cell carcinoma; lymphoepithelial carcinoma; basal cell carcinoma; pilomatrix carcinoma; transitional cell carcinoma; papillary transitional cell carcinoma; adenocarcinoma; gastrinoma, malignant; cholangiocarcinoma; hepatocellular carcinoma; combined hepatocellular carcinoma and cholangiocarcinoma; trabecular adenocarcinoma; adenoid cystic carcinoma; adenocarcinoma in adenomatous polyp; adenocarcinoma, familial polyposis coli; solid carcinoma; carcinoid tumor, malignant; branchiolo-alveolar adenocarcinoma; papillary adenocarcinoma; chromophobe carcinoma; acidophil carcinoma; oxyphilic adenocarcinoma; basophil carcinoma; clear cell adenocarcinoma; granular cell carcinoma; follicular adenocarcinoma; papillary and follicular adenocarcinoma; nonencapsulating sclerosing carcinoma; adrenal cortical carcinoma; endometroid carcinoma; skin appendage carcinoma; apocrine adenocarcinoma; sebaceous adenocarcinoma; ceruminous adenocarcinoma; mucoepidermoid carcinoma; cystadenocarcinoma; papillary cystadenocarcinoma; papillary serous cystadenocarcinoma; mucinous cystadenocarcinoma; mucinous adenocarcinoma; signet ring cell carcinoma; infiltrating duct carcinoma; medullary carcinoma; lobular carcinoma; inflammatory carcinoma; paget's disease, mammary; acinar cell carcinoma; adenosquamous carcinoma; adenocarcinoma w/squamous metaplasia; thymoma, malignant; ovarian stromal tumor, malignant; thecoma, malignant; granulosa cell tumor, malignant; androblastoma, malignant; sertoli cell carcinoma; leydig cell tumor, malignant; lipid cell tumor, malignant; paraganglioma, malignant; extra-mammary paraganglioma, malignant; pheochromocytoma; glomangiosarcoma; malignant melanoma; amelanotic melanoma; superficial spreading melanoma; malig melanoma in giant pigmented nevus; epithelioid cell melanoma; blue nevus, malignant; sarcoma; fibrosarcoma; fibrous histiocytoma, malignant; myxosarcoma; liposarcoma; leiomyosarcoma; rhabdomyosarcoma; embryonal rhabdomyosarcoma; alveolar rhabdomyosarcoma; stromal sarcoma; mixed tumor, malignant; mullerian mixed tumor; nephroblastoma; hepatoblastoma; carcinosarcoma; mesenchymoma, malignant; brenner tumor, malignant; phyllodes tumor, malignant; synovial sarcoma; mesothelioma, malignant; dysgerminoma; embryonal carcinoma; teratoma, malignant; struma ovarii, malignant; choriocarcinoma; mesonephroma, malignant; hemangiosarcoma; hemangioendothelioma, malignant; kaposi's sarcoma; hemangiopericytoma, malignant; lymphangiosarcoma; osteosarcoma; juxtacortical osteosarcoma; chondrosarcoma; chondroblastoma, malignant; mesenchymal chondrosarcoma; giant cell tumor of bone; ewing's sarcoma; odontogenic tumor, malignant; ameloblastic odontosarcoma; ameloblastoma, malignant; ameloblastic fibrosarcoma; pinealoma, malignant; chordoma; glioma, malignant; ependymoma; astrocytoma; protoplasmic astrocytoma; fibrillary astrocytoma; astroblastoma; glioblastoma; oligodendroglioma; oligodendroblastoma; primitive neuroectodermal; cerebellar sarcoma; ganglioneuroblastoma; neuroblastoma; retinoblastoma; olfactory neurogenic tumor; meningioma, malignant; neurofibrosarcoma; neurilemmoma, malignant; granular cell tumor, malignant; malignant lymphoma; hodgkin's disease; hodgkin's; paragranuloma; malignant lymphoma, small lymphocytic; malignant lymphoma, large cell, diffuse; malignant lymphoma, follicular; mycosis fungoides; other specified non-hodgkin's lymphomas; malignant histiocytosis; multiple myeloma; mast cell sarcoma; immunoproliferative small intestinal disease; leukemia; lymphoid leukemia; plasma cell leukemia; erythroleukemia; lymphosarcoma cell leukemia; myeloid leukemia; basophilic leukemia; eosinophilic leukemia; monocytic leukemia; mast cell leukemia; megakaryoblastic leukemia; myeloid sarcoma; and hairy cell leukemia. In further aspects the cancer is a brain cancer (*e.g.,* a glioma), a prostate cancer, a breast cancer (*e.g.,* a triple negative breast cancer), a pancreatic cancer (*e.g.,* a pancreatic ductal adenocarcinoma), acute myeloid leukemia (AML), melanoma, renal cell cancer or chronic lymphocytic leukemia.

In particular aspects, the diseased cell population is a tumor, such as a solid tumor. In further aspects, the primed dendritic cell population is administered to a lymph node that drains the tumor. In specific aspects, the tumor is a metastatic tumor and the primed dendritic cell population is administered to a lymph node that drains the site of the primary tumor. In further aspects the cancer is brain tumor (*e.g.,* a glioma), a prostate tumor, a breast tumor (*e.g.,* a triple negative breast cancer), a pancreatic tumor (*e.g.,* a pancreatic ductal adenocarcinoma) or a renal cell tumor.

In further aspects, the embodiments may further comprise administering a composition of the present invention more than one time to the subject, such as, for example, 1, 2, 3, 4, 5, 6, 7, 8, 9, 10, 15, 20 or more times.

A subject for treatment according to the embodiments is, in some aspects, a mammalian subject. For example, the subject may be a primate, such a human. In further aspects, the subject is a non-human mammal, such as a dog, cat, horse, cow, goat, pig or zoo animal.

Further disclosed for reference is an immunogenic composition comprising: (i) an antigen-primed dendritic cell and (ii) a Type I interferon (INF), a TLR-7 agonist, a TLR-9 agonist, AIMp1, a TLR-3 agonist, a retinoic acid inducible gene-1 (RIG-1)-like receptor ligand or a cytosolic DNA (CDS) receptor ligand. The antigen-primed dendritic cell may have been primed with an antigen associated with a cancer, an autoimmune disease or an infectious disease. Furthermore, the antigen-primed dendritic cell may have been primed with at least one tumor antigen.

Thus, a composition disclosed herein may comprise a primed dendritic cell population and a Type I INF. The Type I INF may be INF-α, IFN-β, IFN-ε, IFN-κ or IFN-ω. Furthermore, a composition may comprise a primed dendritic cell population and a TLR-7 agonist. The TLR-7 agonist may be selected from the group consisting of CL075, CL097, CL264, CL307, GS-9620, Poly(dT), imiquimod, gardiquimod, resiquimod (R848), loxoribine, and a ssRNA oligonucleotide. Furthermore, a composition may comprise a primed dendritic cell population and a TLR-9 agonist. In some cases, the TLR-9 agonist may be a CpG oligodeoxynucleotide (CpG ODN). Furthermore, a composition disclosed herein may comprise a primed dendritic cell population and AIMp1.

Further disclosed for reference is an immunogenic composition comprising: (i) an antigen-primed dendritic cell and (ii) a TLR-3 agonist, RIG-1-like receptor ligand, or CDS receptor ligand. The antigen-primed dendritic cell may have been primed with an antigen associated with a cancer, an autoimmune disease or an infectious disease. In certain aspects, the antigen-primed dendritic cell has been primed with at least one tumor antigen. In some aspects, the tumor is a brain tumor, renal cell cancer, melanoma, prostate cancer, breast cancer, or chronic lymphocytic leukemia. In some aspects, the composition comprises a TLR-3 agonist. The TLR-3 agonist may be Poly(I:C) or RGC100. Furthermore, the composition may comprise a RIG-1-like receptor ligand. The RIG-1-like receptor ligand may be selected from the group consisting of a MDA5 ligand, a LGP2 ligand, a ssRNA, a dsRNA, 5'ppp-dsRNA, Poly(dA:dT), and Poly(I:C). Furthermore, the composition may comprise a CDS receptor ligand. For example, the CDS receptor ligand is bacterial CDNs.

Further disclosed for reference is a method for culturing antigen specific T-cells, comprising culturing a population of T-cells or T-cell precursors in the presence of an antigen presenting cell population, wherein said culturing is in the presence of AIMp1. The antigen presenting cell population may be a primed dendritic cell population. Furthermore, the antigen presenting cell population can be artificial antigen presenting cells, such cells that have been inactivated (*e*.*g*., by irradiation). Furthermore, the method may further defined as a method for *ex vivo* expansion of antigen specific T-cells. The dendritic cell population may comprise primary dendritic cells. Furthermore, said culturing may be in the presence of an immune checkpoint inhibitor, such as a CTLA-4 antagonist. In specific aspects, the CTLA-4 antagonist is an inhibitor nucleic acid specific to CTLA-4. In certain aspects, the inhibitory nucleic acid is a RNA. In further aspects, the RNA is a small interfering RNA (siRNA) or a short hairpin RNA (shRNA). In other aspects, the CTLA-4 antagonist is a CTLA-4-binding antibody.

Further disclosed for reference is a method of culturing antigen specific T-cells comprising culturing a population of T-cells or T-cell precursors in the presence of a population of antigen presenting cells that have been primed with at least a first antigen, wherein said culturing is in the presence of Poly(I:C). The method may be further defined as a method for ex vivo expansion of antigen specific T-cells. The antigen presenting cells may comprise dendritic cells. In certain aspects, the dendritic cells are homologously loaded with antigen. In some aspects, the dendritic cell population comprises primary dendritic cells. Furthermore, the culturing may be in the presence of an immune checkpoint inhibitor to the subject. In some aspects, the immune checkpoint inhibitor is a CTLA-4 antagonist. In particular aspects, the immune checkpoint inhibitor is ipilimumab, pembrolizumab or nivolumab.

As used herein, "essentially free," in terms of a specified component, is used herein to mean that none of the specified component has been purposefully formulated into a composition and/or is present only as a contaminant or in trace amounts. The total amount of the specified component resulting from any unintended contamination of a composition is therefore well below 0.05%. Most preferred is a composition in which no amount of the specified component can be detected with standard analytical methods.

As used herein in the specification and claims, "a" or "an" may mean one or more. As used herein in the specification and claims, when used in conjunction with the word "comprising", the words "a" or "an" may mean one or more than one. As used herein, in the specification and claim, "another" or "a further" may mean at least a second or more.

As used herein in the specification and claims, the term "about" is used to indicate that a value includes the inherent variation of error for the device, the method being employed to determine the value, or the variation that exists among the study subjects.

Other objects, features and advantages of the present invention will become apparent from the following detailed description. It should be understood, however, that the detailed description and the specific examples, while indicating certain embodiments of the invention, are given by way of illustration only.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.

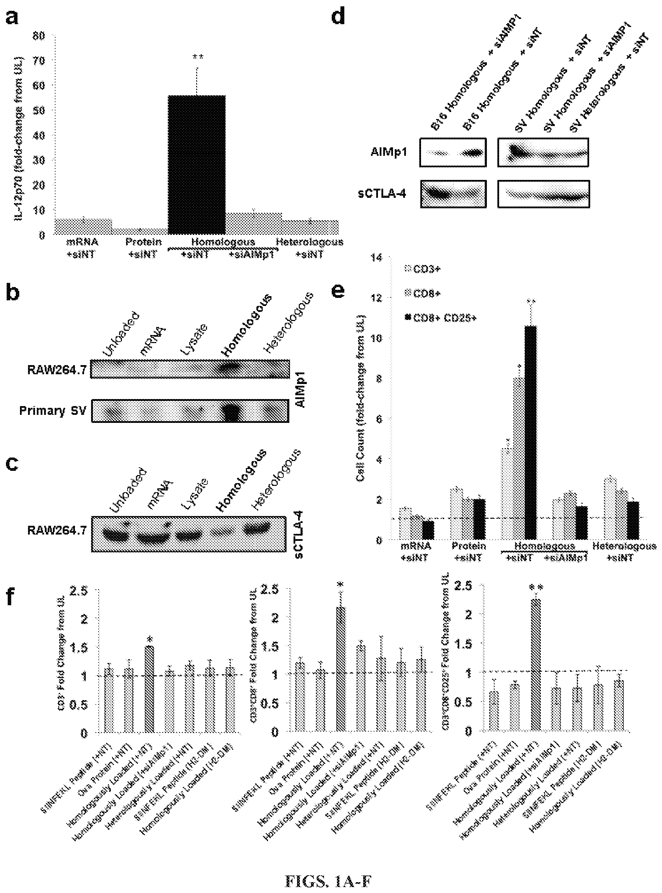

**FIGS. 1A****-F:** Concurrent loading of antigenically related MHC Class I and II determinants promote T_{H}1-polarization of mouse DC. **(A)** Mouse DC loaded in tandem with homologous mRNA (electroporation) and lysate (incubation) secreted significantly more IL-12p70 than alternatively loaded DC when co-cultured with splenocytes as assayed by ELISA. This effect was abrogated by siRNA knockdown of AIMp1. Antigens were derived from the RAW264.7 cell line. Heterologous Class II antigens were derived from the 4T1 breast cancer cell line. Y-axis: fold change in IL-12p70 secretion from unloaded (UL) DC. **(B)** Prior to co-culture, homologously loaded DC secreted significantly more AIMp1 than DC loaded with heterologous determinants or otherwise loaded singly as assayed by Western blot of 48 hour cell culture supernatants. Top panel: AIMp1 release following homologous loading with RAW264.7 antigenic determinants. Heterologous lysate derived from 4T1. Bottom panel: AIMp1 release following homologous loading with primary SV antigenic determinants. Heterologous lysate derived from primary prostate. **(C)** Homologously loaded DC also displayed a significant decrease in sCTLA-4 secretion. Shown: sCTLA-4 release following homologous loading with RAW264.7 antigenic determinants. Heterologous lysate derived from 4T1. **(D)** AIMp1 siRNA knockdown in homologously-loaded DC restored sCTLA-4 secretion to baseline from DC homologously loaded with B16 melanoma determinants (left panels) and primary SV determinants (right panels). Data shown are indicative of typical AIMp1 knockdown of > 70%. **(E)** DC homologously-loaded with SV determinants generated significantly increased CD3⁺, CD3⁺CD8⁺, and CD3⁺CD8⁺CD25⁺ T-cell proliferation during *in vitro* co-culture with autologous splenocytes in comparison to DC loaded by any other fashion. This effect was abrogated by treatment of homologously-loaded DC with AIMp1 siRNA. Heterologous lysate was primary prostate. Y-axis: fold change in total cell count in comparison to unloaded (UL) DC. **(F)** DC homologously-loaded with SIINFEKL (SEQ ID NO: 1) MHC Class I H-2K^{d} binding epitope and whole OVA protein generated significantly increased CD3⁺, CD3⁺CD8⁺, and CD3⁺CD8⁺CD25⁺ T-cell proliferation *in vivo* in comparison to DC loaded by any other fashion. This effect was abrogated by treatment of homologously-loaded DC with AIMp1 siRNA and was not observed in H2-DM^{-/-} DC. Heterologous Class II antigen was primary SV lysate. Y-axes: percent fold change in comparison to unloaded (UL) DC. For all experiments siNT or NT = non-targeting siRNA. siAIMp1 = AIMp1 siRNA.

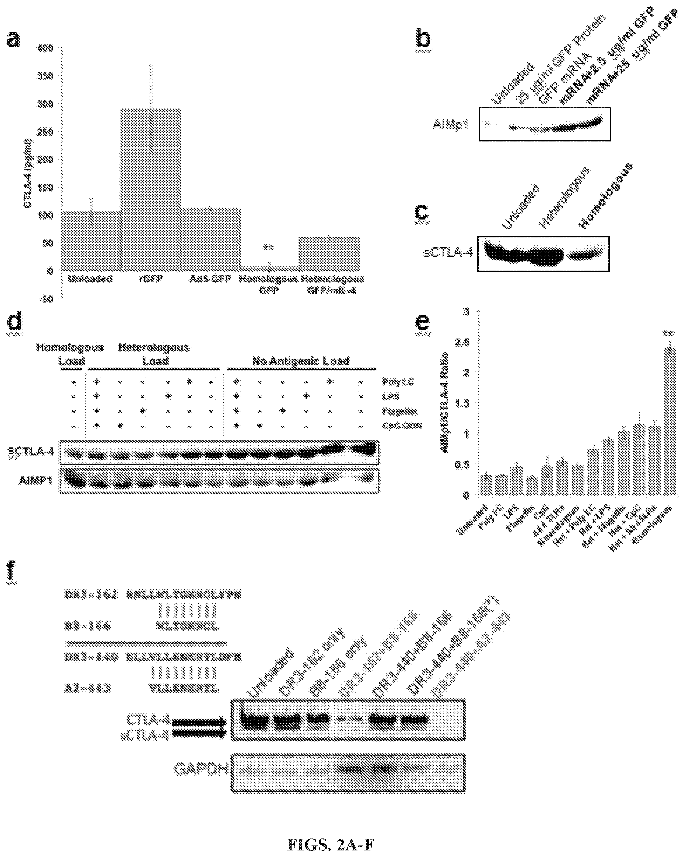

**FIGS. 2A****-F:** Homologous Class I and II antigenic determinants generate T_{H}1-polarized DC in a manner independent of PRR agonism. **(A)** sCTLA-4 ELISA of human DC culture supernatants following transduction with GFP-expressing adenovirus, incubation with rGFP protein, neither, or both. Heterologous loading was represented as well using an irrelevant antigen, murine IL-4. **(C)** AIMp1 Western blot of human DC culture supernatants following electroporation with GFP-mRNA, incubation with rGFP protein, neither, or both at two different concentrations of protein. One representative experiment of five shown. **(C)** sCTLA-4 Western blot of human DC culture supernatants following electroporation with class I influenza HA peptide and incubation with overlapping (homologous) or nonoverlapping (heterologous) class II influenza HA peptide. **(D)** sCTLA-4 and AIMp1 western blots of human DC culture supernatants following loading with homologous or heterologous class I and II peptide pairs in the presence of various TLR agonistic stimuli. Representative experiment of three shown. **(E)** Densitometry quantitation of AIMp1/sCTLA-4 ratios of the data depicted in (d) over three independent experiments **(F)** RT-PCR analysis indicates loading of DC with homologous influenza HA peptide pairs (B8-166 (SEQ ID NO: 3)/DR3-162 (SEQ ID NO: 2) and A2-443 (SEQ ID NO: 5)/DR3-440 (SEQ ID NO: 4)) (Decker *et al.,* 2009) significantly downregulated CTLA-4 and sCTLA-4 mRNA transcripts. Unloaded, singly-loaded, and heterologously-loaded DC continued to express CTLA-4. GAPDH amplification shown as load control. (*) = loading of heterologous peptide pairs +/electroporation of the class I peptide had no effect upon CLTA-4 expression.

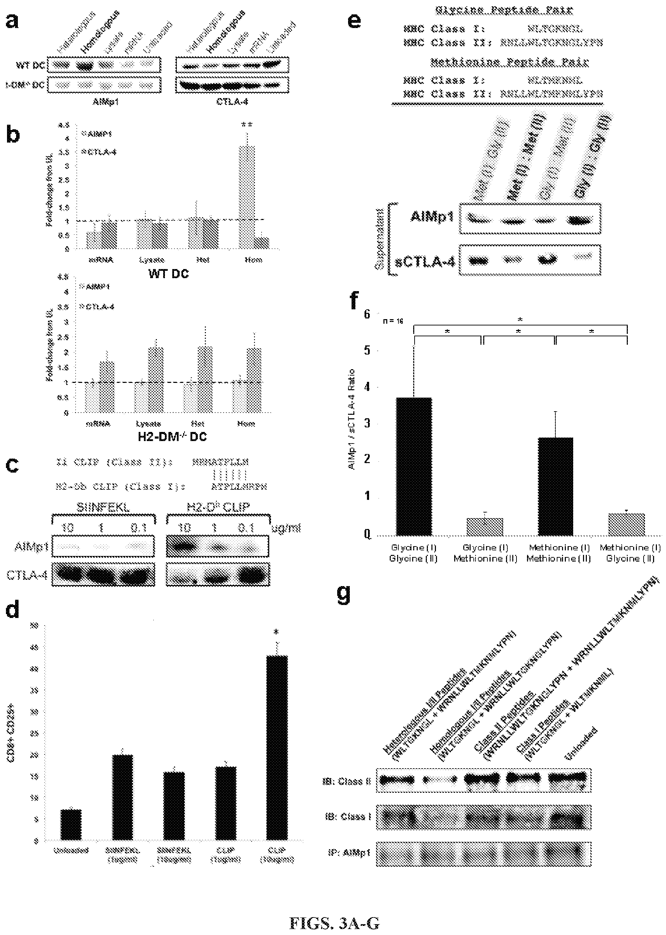

**FIGS. 3A****-G:** AIMp1/sCTLA-4 modulation is dependent upon binding of homologous class I and II peptide to MHC. **(A)** sCTLA-4 (right) and AIMp1 (left) western blots of wild type (top) and H2-DM^{-/-} (bottom) murine DC culture supernatants following single, homologous, or heterologous loading with SV mRNA and lysate. Representative experiment shown. **(B)** Densitometry quantitation of AIMp1/sCTLA-4 ratios of the data depicted in (a) over three independent experiments. Top panel: wild type DC. Bottom panel: H2-DM^{-/-} DC. (C) Loading of H2-DM^{-/-} DC with a class I H-2D^{b} peptide possessing amino acid sequence homology to CLIP recapitulates appropriate regulation of AIMp1 and sCTLA-4 (Ii CLIP = SEQ ID NO: 6; H2-Db CLIP = SEQ ID NO: 7). **(D)** High AIMp1/sCTLA-4 secretion ratio in response to homologous loading of H2-DM^{-/-} DC with class I H-2D^{b} CLIP mediated downstream augmentation of a T_{H}1 response *in vitro* as evidenced by enhanced development of activated CD8⁺ T-cells following coculture of H-2D^{b} CLIP-loaded H2-DM^{-/-} DC with autologous splenocytes. **(E)** Amino acid substitutions (Gly to Met) introduced into either the class I or the class II binding peptide, thereby limiting regions of contiguous homology to three amino acids, were sufficient to abolish high AIMp1/sCTLA-4 secretory ratios in response to peptide loading. Introduction of the compensatory substitutions on the cognate peptide, thereby recapitulating extended homology, was sufficient to rescue high AIMp1/sCTLA-4 secretory ratios. Shown: Representative experiment of 16. (Glycine Pair: Class I = SEQ ID NO: 3, Class II = SEQ ID NO: 2; Methionine Pair: Class I = SEQ ID NO: 8, Class II = SEQ ID NO: 9) **(F)** Densitometric quantitation of the data presented in (e). Average values of 16 independent experiments. *p < 0.05. **(G)** AIMp1 coIP indicated that interaction of AIMp1 with MHC was substantially abolished when DC were loaded with homologous peptides, suggesting secreted AIMp1 might be derived from MHC-bound AIMp1. (Heterologous Peptides = SEQ ID NO: 3 & SEQ ID NO: 10; Homologous Peptides = SEQ ID NO: 3 & SEQ ID NO: 11; Class II Peptides = SEQ ID NO: 11 & SEQ ID NO: 10; Class I Peptides = SEQ ID NO: 3 & SEQ ID NO: 8)

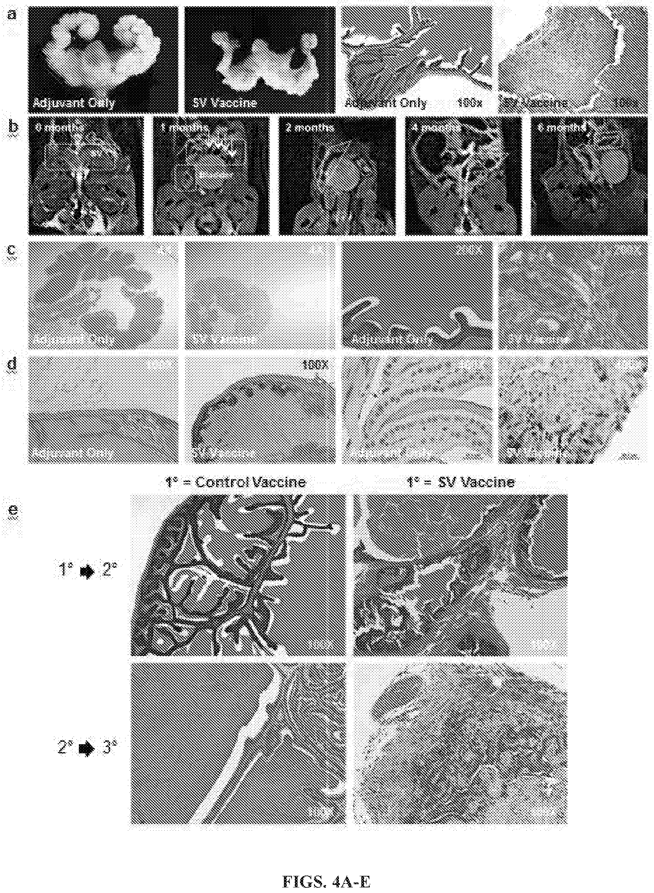

**FIGS. 4A****-E:** DC polarized by homologous antigenic determinants overcome peripheral tolerance and ablate the seminal vesicle. **(A)** Gross (right) and sub-gross (left) images of seminal vesicle taken from male C57BL/6 mice injected with adjuvant only or SV-loaded homologous DC vaccine plus adjuvant. **(B)** 3D longitudinal MRI demonstrating ablation of the SV over a time course of six months. **(C)** H&E stained SV at six months post-vaccination demonstrating vestigial SV comprised primarily of fibrosis in vaccinated mice in comparison to mice that received adjuvant only. **(D)** At one month post-treatment, IHC staining with anti-CD8 indicates significant CD8⁺ T-cell infiltration in vaccinated mice in comparison to mice that received adjuvant only. **(E)** Adoptive transfer of peripheral blood lymphocytes (PBL) from reactive SV-vaccinated animals into naive recipients recapitulated SV destruction within six weeks of transfer. Adoptive transfer of PBL from sham-vaccinated or adjuvant-only animals exhibited no effect upon normal SV histopathology (top panels). Secondary adoptive transfers produced the same effect (bottom panels). Total SV reactivity = 25/42 (60%).

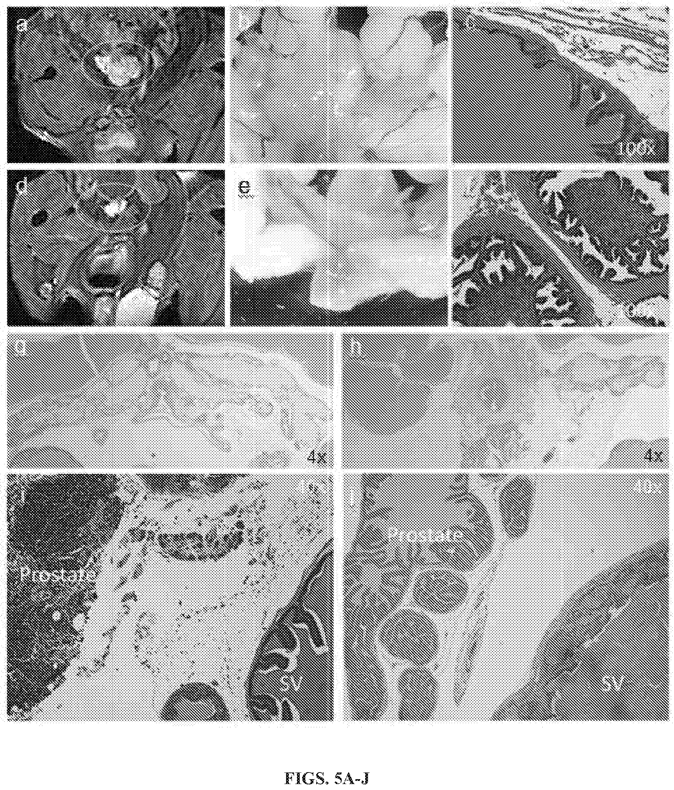

**FIGS. 5A****-J:** DC polarized by homologous antigenic determinants ablate the prostate and the seminal vesicle in a specific and differential fashion. MRI **(A & D)** and gross **(B & E)** images of normal prostate (top) and prostate derived from animals vaccinated with homologously-loaded DC prostate vaccine (bottom). H&E staining of vaccinated and adjuvant-only animals indicates **(C)** normal anterior lobe in adjuvant-only animal and **(F)** distinct hypertrophy and inflammation in the anterior lobe of the vaccinated animal. Over time, prostatic lobes in vaccinated animals tended to shrink and disappear as indicated by image of absent and hypoplastic anterior lobe of vaccinated animal **(H)** in comparison to adjuvant-only animal **(G).** Though SV and prostate are in close proximity, vaccination was highly specific as demonstrated by **(I)** inflammatory pathology of prostate in conjunction with normal SV in prostate-vaccinated animal as well as **(J)** characteristic inflammatory pathology of SV in conjunction with normal prostate in SV-vaccinated animal. Total prostate reactivity = 8/17 (47%).

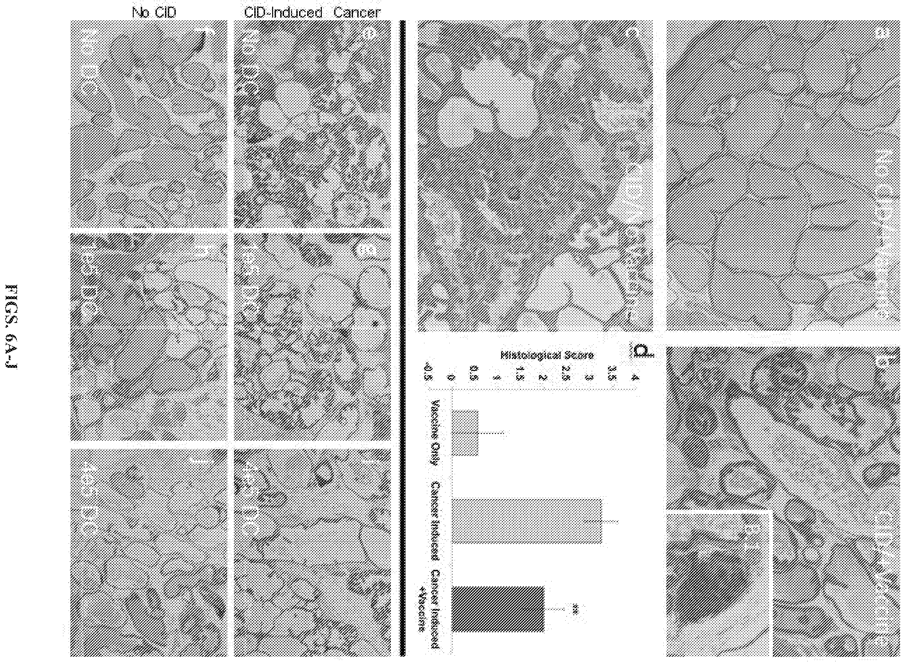

**FIGS. 6A****-J:** Homologous vaccination discerns normal from neoplastic self and controls prostatic adenocarcinoma in a physiologic model system. Prostatic adenocarcinoma determinants derived from Pro-Cat/JOCK1 mice (Carstens *et al.,* 2014) were used to vaccinate additional cohorts of Pro-Cat/JOCK1 animals at the stage of adenocarcinoma. The data indicated that **(A)** while adenocarcinoma-loaded vaccine and adjuvant had little evident cross-reactivity with normal prostate, **(B)** mice induced to the adenocarcinoma stage of disease exhibited pathologic findings most reminiscent of mPIN when vaccinated and also exhibited lymphocyte-infiltrated acini **(B.1 inset)** not seen in unvaccinated or non-cancerous mice. Induced mice receiving adjuvant only **(C)** displayed typical pathologic characteristics of Pro-Cat/JOCK1 adenocarcinoma. **(D)** Pathological scoring (as defined in experimental procedures) indicated a statistically significant difference in pathological phenotype between vaccinated induced mice and induced mice treated with adjuvant only. Y-axis: histopathologic score. Vaccination also appeared to impart a dose responsive effect as observed among induced **(E, G, I)** and uninduced control animals **(F, H, J)** that received no DC **(E & F),** 1 x 10⁵ DC **(G & H),** and 4 x 10⁵ DC **(I & J).** Reference bar = 100 µM.

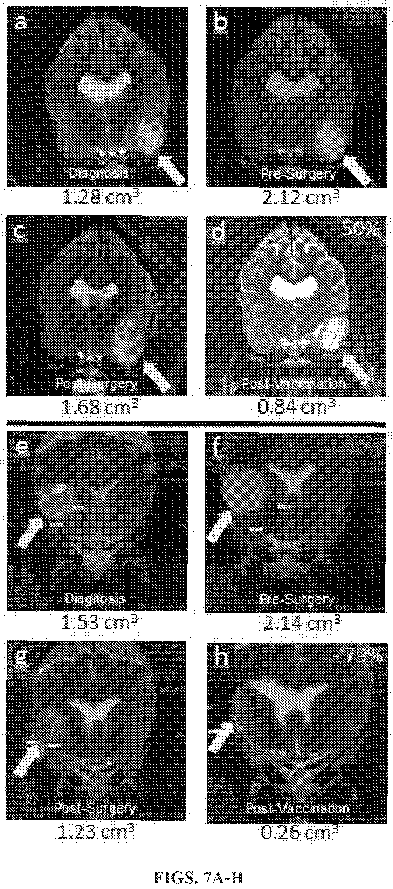

**FIGS. 7A****-H:** Homologous vaccination is feasible and of potential efficacy in a large animal model of spontaneous CNS malignancy. Upon diagnosis of CNS malignancy, two large (> 25 kg) canine patients were recruited for a non-randomized phase I veterinary trial. Clinical MR imaging of the brain shows tumors of two animals at diagnosis **(A & E),** immediately prior to conservative tumor resection **(B & F),** immediately after resection **(C & G),** and five weeks after the initiation of the vaccination protocol **(D & H).** Arrows indicate the region of the tumor. The first animal (top four panels) exhibited a reduction in tumor volume of 50% after a single vaccine injection (see **C & D)** whereas the second animal (bottom four panels) exhibited a reduction in tumor volume of 79% (see **G & H)** after three vaccine injections.

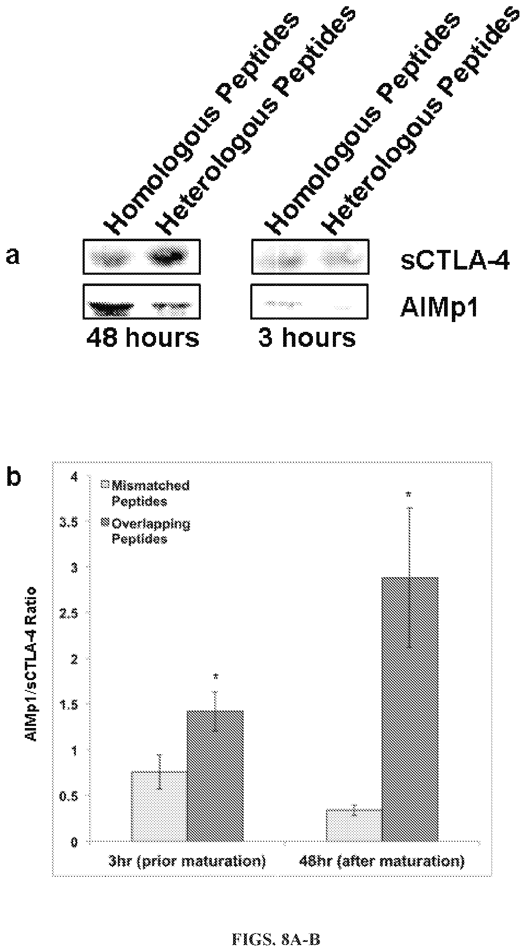

**FIGS. 8A****-B:** AIMp1 is released early and prior to maturation following homologous loading of DC whereas ablation of sCTLA-4 secretion occurs downstream. **(A)** Initiation of AIMp1 secretion was observed as early as three hours after DC loading with homologous Class I and II peptides, whereas sCTLA-4 secretion remained unaffected at this early time point. Ablation of sCTLA-4 secretion was not seen until later time points post maturation (e.g. as shown 48 hours after loading). **(B)** Quantitation of AIMp1 and sCTLA-4 secretion at three hours and 48 hours post-loading from DC loaded with mismatched heterologous peptides (light gray) or overlapping homologous peptides (dark gray). Y-axis: AIMp1/sCTLA-4 ratio.

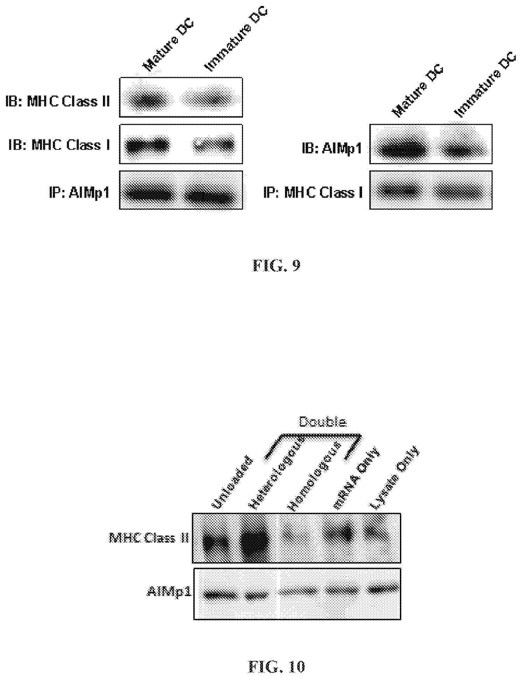

**FIG. 9****:** AIMp1 coIP and reciprocal MHC class I coIP indicate strong interaction, especially in mature DC, between AIMp1 and MHC. Results were confirmed by tandem mass spectroscopy (not shown). IP = specificity of antibody used for immunoprecipitation. IB = specificity of antibody used for detection. Representative experiment of three shown.
**FIG. 10****:** AIMp1 binding to MHC is dependent upon loading of DC with homologous antigenic determinants (mRNA and lysate). In a manner similar to that of DC loaded with homologous class I and II peptide pairs, AIMp1 coIP indicated that interaction of AIMp1 with MHC class II and class I (not shown) was substantially abolished when DC were loaded with homologous mRNA and lysate preparations, suggesting that secreted AIMp1 might be derived from MHC-bound AIMp1. Interestingly, introduction of increased antigenic heterogeneity between class I (mRNA) and class II (lysate) determinants by use of heterologous preparations appeared to enhance AIMp1 binding to MHC. Representative experiment of three shown.

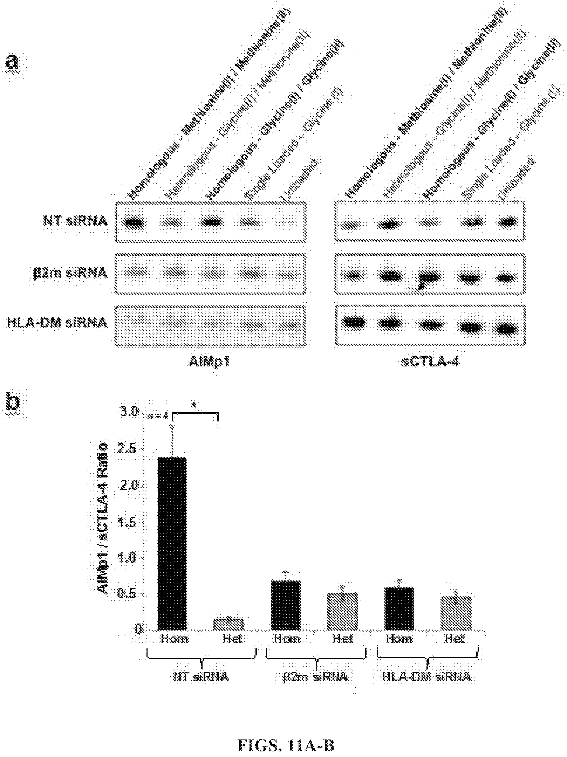

**FIGS. 11A****-B:** Abrogation of MHC loading by siRNA knockdown of β2-microglobulin or HLA-DM eliminates the ability of human DC to regulate AIMp1/sCTLA-4 secretion in response to homologous peptide loading. **(A)** DC loaded with two different pairs of homologous peptides (wild type influenza HA and gly-to-met substituted HA) augmented AIMp1 secretion and diminished sCTLA-4 secretion when treated with non-targeting (NT) siRNA; however, treatment with either β2-microglobulin or HLA-DM siRNA completed abrogated the ability to regulate AIMp1 and sCTLA-4, rendering secreted AIMp1/sCTLA-4 ratios indistinguishable from those of unloaded, singly-loaded, or heterologously-loaded DC. Representative of four experiments shown. **(B)** Densitometric quantitation of the data presented in (a). Average values of four independent experiments. *p < 0.05.

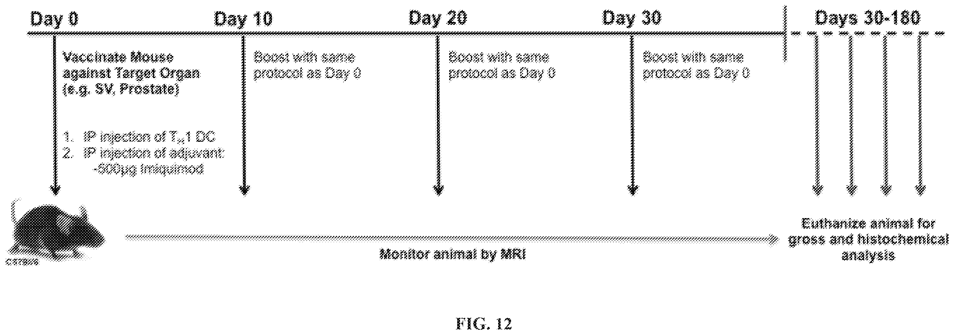

**FIG. 12****:** Schematic outlining methodology of DC homologous loading for *in vivo* targeting of immunologic self.

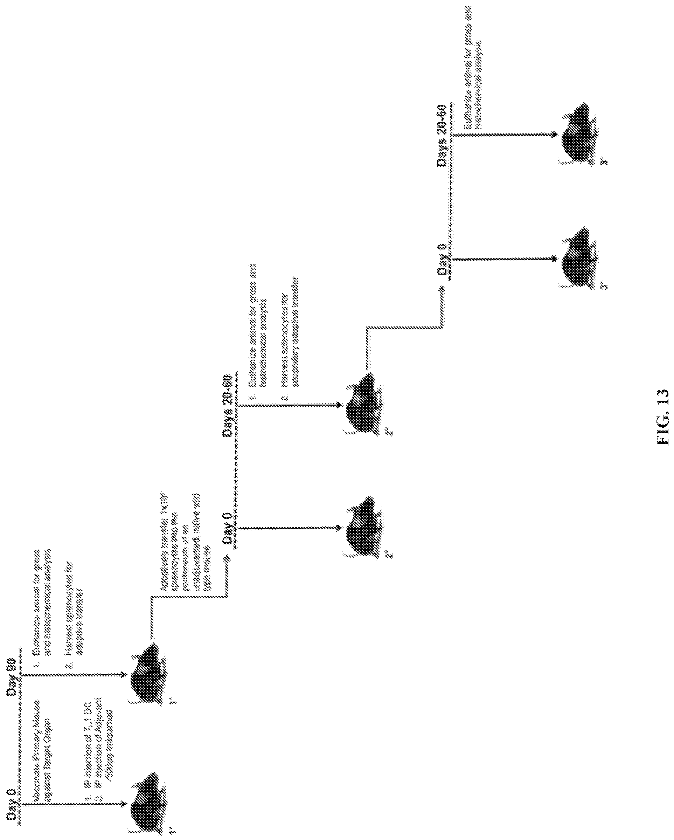

**FIG. 13****:** Schematic outlining adoptive transfer schedule and analysis timelines.

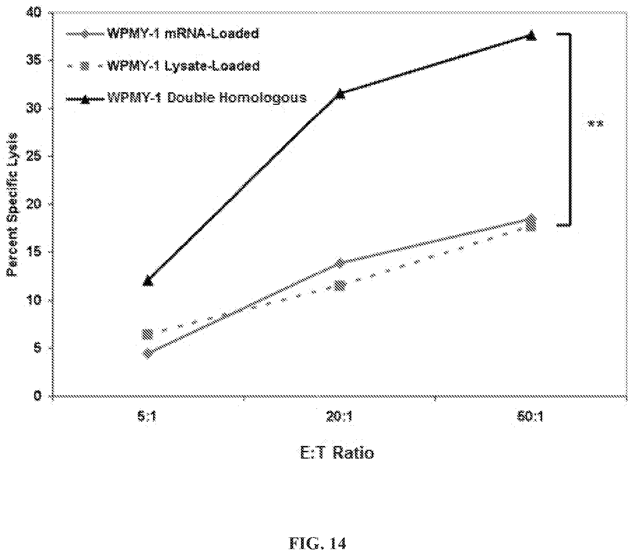

**FIG. 14****:** Homologous loading of partially-HLA matched human DC generates CTL with enhanced ability to specifically lyse WPMY-1 normal prostate. Human DC loaded with determinants derived from the WPMY-1 normal human prostate cell line were used to prime and expand autologous lymphocytes. After three stimulations, T-cells were harvested and lytic specificity tested by ⁵¹Cr lysis assay, performed as described previously.⁵⁰ The data indicated that DC loaded with both WPMY-1 mRNA and WPMY-1 lysate generated substantially superior specific CTL activity than DC loaded with either WPMY-1 lysate or WPMY-1 mRNA alone. Y-axis: Percent specific lysis. X-axis: E:T ratio.

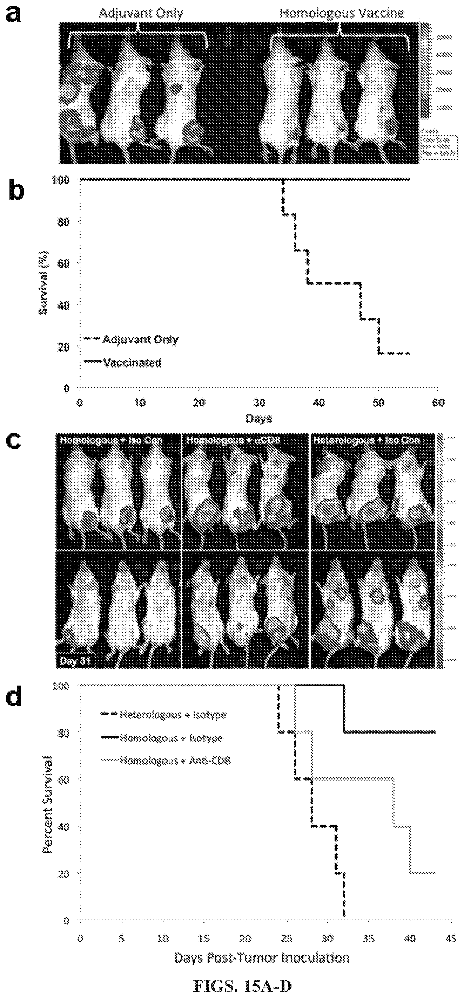

**FIGS. 15A****-D:** Vaccines generated by homologous antigenic loading inhibit growth and metastatic spread of established 4T1 breast cancer tumors in a manner dependent upon CD8⁺ cells. (A) Eight days after ectopic establishment of luciferase-expressing 4T-1 tumors, mice were vaccinated with homologously-loaded dendritic cells or treated with sham vaccination and systemic adjuvant. Metastatic spread of the tumors was monitored by IVIS. As shown, sham-vaccinated mice developed distant metastases to liver, lungs, and brain and began to die at one month post-implantation. Vaccinated mice did not develop metastatic disease and primary tumors remained well-controlled. Antigenic materials for homologous loading were derived from a 4T1 cell line that did **NOT** express luciferase. Representative mice shown. Cohort size = 6 per group. (B) Kaplan-Meier survival analysis of the data depicted by (A). p < 0.001 at day 56. (C) To demonstrate dependency upon CD8⁺ cells, cohorts of non-tumor-bearing mice were vaccinated twice with homologous vaccine. Following CD8-depletion of a single cohort, splenocytes were harvested and adoptively transferred into cohorts of tumor-bearing mice. The data demonstrated that, while mice adoptively-transferred with homologously-vaccinated, isotype-depleted splenocytes maintained tumor control and did not exhibit metastatic spread, mice adoptively-transferred with homologously-vaccinated, CD8-depleted splenocytes, ultimately succumbed to disease. Mice adoptively-transferred with heterologously-vaccinated, isotype-depleted splenocytes fared worst of all, indicating perhaps the presence of additional, CD8⁻ effectors generated by homologous vaccination. Representative mice shown. Cohort size = 5 per group. (D) Kaplan-Meier survival analysis of the data depicted by (C). On day 43, 80% of mice adoptively transferred with isotype-depleted splenocytes derived from homologously-vaccinated hosts remained alive versus only 20% of mice adoptively transferred with isotype-depleted splenocytes derived from homologously-vaccinated hosts. All mice adoptively transferred with splenocytes derived from isotype-depleted, heterologously-vaccinated hosts had succumbed to tumor by day 32. p<0.001 at day 43.

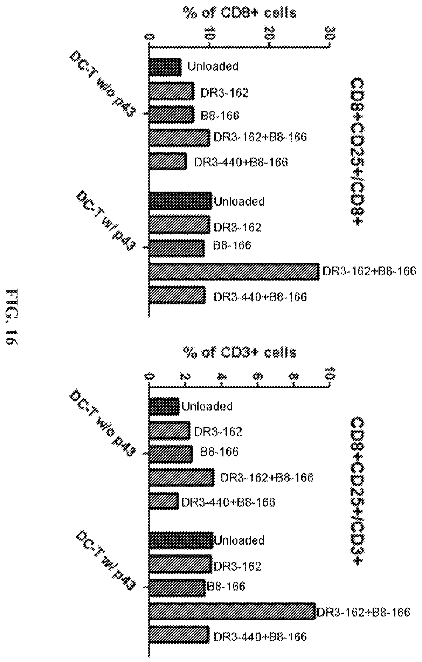

**FIG. 16****:** Recombinant AIMp1 (p43) enhances the generation of T_{H}1 responses. Human DC were loaded with the indicated peptide combinations and used to prime autologous T-cells in either the presence or absence of 100 ng/ml recombinant AIMp1 (p43). Under all conditions tested, the percentage of activated CD8+ T-cells was enhanced 50% to 150% in the presence of AIMp1. p<0.05 for each paired condition tested. The most robust increase between rAIMp1 was observed with DC that were homologously-loaded with antigen.

### DESCRIPTION OF ILLUSTRATIVE EMBODIMENTS

### I. The Present Embodiments

Dendritic cells comprise a highly specialized class of antigen presenting cells. Previous studies have demonstrated that dendritic cells can be effectively primed to stimulate a T-cell response that is specifically targeted to a cell population in in subject, such as cancer cell (see, *e.g.,* U.S. Patent 8,728,806). However, there remained a need for methods to enhance the efficacy of dendritic cell compositions and thereby provide a more robust immune response.

Studies presented herein demonstrate for the first time that both co-stimulator molecules and the site of dendritic cell application can be significantly effect the efficacy of the cell compositions. In particular, the administration of dendritic cells in conjunction with a co-stimulator such as Type I interferon (*e*.*g*., INFα), a TLR-7 agonist, a TLR-9 agonist, or AIMp1 was found to significantly enhance the T-cell response provided by primed dendritic cell compositions. Likewise, enhanced T-cell responses could be achieved with primed dendritic cells administered in conjunction with a TLR-3 agonist, a retinoic acid inducible gene-1 (RIG-1)-like receptor ligand or a cytosolic DNA (CDS) receptor ligand. Moreover, the site of application of dendritic cells was found to significantly vary the effectiveness of T-cell stimulation. In particular, without being bound by any particular theory of the mechanism of action, it is believed that the dendritic cells should preferably be exposed to T-cells that are proximal to the site to the targeted disease tissue (*e*.*g*., tumor). Accordingly, in some preferred aspects, dendritic cell compositions are administered to a subject by direct introduction into lymphoid tissue that drains the site of a diseased cell population, such as a tumor. Thus, by combined use of co-stimulator molecules and administration of primed dendritic cells to a site proximal to disease tissue an extremely robust immune response can be induced.

### II. Dendritic Cell Populations of the Embodiments

Methods for isolating culturing and priming dendritic cells are well known in the art. For example, U.S. Patent 8,728,806 provides detailed methods for providing antigen primed dendritic cells that may be used in the compositions and methods disclosed herein. In certain aspects, dendritic cells for use according to the embodiments are isolated from a subject that is to be treated according to the embodiments. In other aspects, dendritic cells may be from a different subject, such as an HLA-matched donor. In certain aspects, the dendritic cells are from a bank of dendritic cells having a defined HLA typing. In preferred aspects, primed dendritic cells for use according to the embodiments are homologously-loaded with antigen as detailed herein and in U.S. Patent 8,728,806.

Methods for isolating cell populations enriched for dendritic cell precursors and immature dendritic cells from various sources, including blood and bone marrow, are known in the art. For example, dendritic cell precursors and immature dendritic cells can be isolated by collecting heparinized blood, by apheresis or leukapheresis, by preparation of buffy coats, rosetting, centrifugation, density gradient centrifugation (*e*.*g*., using Ficoll (such as FICOLL-PAQUE^{®}), PERCOLL^{®} (colloidal silica particles (15-30 mm diameter) coated with non-dialyzable polyvinylpyrrolidone (PVP)), sucrose, and the like), differential lysis of cells, filtration, and the like. A leukocyte population can be prepared, such as, for example, by collecting blood from a subject, defribrinating to remove the platelets and lysing the red blood cells. Dendritic cell precursors and immature dendritic cells can optionally be enriched for monocytic dendritic cell precursors by, for example, centrifugation through a PERCOLL^{®} gradient. Furthermore, dendritic cell precursors can be selected using CD14 selection of G-CSF mobilized peripheral blood.

Dendritic cell precursors and immature dendritic cells optionally can be prepared in a closed, aseptic system. As used herein, the terms "closed, aseptic system" or "closed system" refer to a system in which exposure to non-sterilize, ambient, or circulating air or other non-sterile conditions is minimized or eliminated. Closed systems for isolating dendritic cell precursors and immature dendritic cells generally exclude density gradient centrifugation in open top tubes, open air transfer of cells, culture of cells in tissue culture plates or unsealed flasks, and the like. The closed system may typically allow aseptic transfer of the dendritic cell precursors and immature dendritic cells from an initial collection vessel to a sealable tissue culture vessel without exposure to non-sterile air.

Monocytic dendritic cell precursors may be isolated by adherence to a monocyte-binding substrate. For example, a population of leukocytes (e*.g*., isolated by leukapheresis) can be contacted with a monocytic dendritic cell precursor adhering substrate. When the population of leukocytes is contacted with the substrate, the monocytic dendritic cell precursors in the leukocyte population preferentially adhere to the substrate. Other leukocytes (including other potential dendritic cell precursors) exhibit reduced binding affinity to the substrate, thereby allowing the monocytic dendritic cell precursors to be preferentially enriched on the surface of the substrate.

Suitable substrates include, for example, those having a large surface area to volume ratio. Such substrates can be, for example, a particulate or fibrous substrate. Suitable particulate substrates include, for example, glass particles, plastic particles, glass-coated plastic particles, glass-coated polystyrene particles, and other beads suitable for protein absorption. Suitable fibrous substrates include microcapillary tubes and microvillous membrane. The particulate or fibrous substrate usually allows the adhered monocytic dendritic cell precursors to be eluted without substantially reducing the viability of the adhered cells. A particulate or fibrous substrate can be substantially non-porous to facilitate elution of monocytic dendritic cell precursors or dendritic cells from the substrate. A "substantially non-porous" substrate is a substrate in which at least a majority of pores present in the substrate are smaller than the cells to minimize entrapping cells in the substrate.

Adherence of the monocytic dendritic cell precursors to the substrate can optionally be enhanced by addition of binding media. Suitable binding media include monocytic dendritic cell precursor culture media (*e*.*g*., AIM-V^{®}, RPMI 1640, DMEM, X-VIVO 15^{®}, and the like) supplemented, individually or in any combination, with for example, cytokines (*e*.*g*., Granulocyte/Macrophage Colony Stimulating Factor (GM-CSF), Interleukin 4 (IL-4), or Interleukin 13 (IL-13)), blood plasma, serum (*e*.*g*., human serum, such as autologous or allogenic sera), purified proteins, such as serum albumin, divalent cations (*e*.*g*., calcium and/or magnesium ions) and other molecules that aid in the specific adherence of monocytic dendritic cell precursors to the substrate, or that prevent adherence of non-monocytic dendritic cell precursors to the substrate. The blood plasma or serum can be heated-inactivated. The heat-inactivated plasma can be autologous or heterologous to the leukocytes.

Following adherence of monocytic dendritic cell precursors to the substrate, the non-adhering leukocytes are separated from the monocytic dendritic cell precursor/substrate complexes. Any suitable means can be used to separate the non-adhering cells from the complexes. For example, the mixture of the non-adhering leukocytes and the complexes can be allowed to settle, and the non-adhering leukocytes and media decanted or drained. Alternatively, the mixture can be centrifuged, and the supernatant containing the non-adhering leukocytes decanted or drained from the pelleted complexes.

Isolated dendritic cell precursors can be cultured *ex vivo* for differentiation, maturation and/or expansion. (As used herein, isolated immature dendritic cells, dendritic cell precursors, T cells, and other cells, refers to cells that, by human hand, exists apart from their native environment, and are therefore not a product of nature. Isolated cells can exist in purified form, in semi-purified form, or in a non-native environment.) Briefly, *ex vivo* differentiation typically involves culturing dendritic cell precursors, or populations of cells having dendritic cell precursors, in the presence of one or more differentiation agents. Suitable differentiating agents can be, for example, cellular growth factors (*e*.*g*., cytokines such as (GM-CSF), Interleukin 4 (IL-4), Interleukin 13 (IL-13), and/or combinations thereof). In certain embodiments, the monocytic dendritic cells precursors are differentiated to form monocyte-derived immature dendritic cells.

The dendritic cell precursors can be cultured and differentiated in suitable culture conditions. Suitable tissue culture media include AIM-V^{®}, RPMI 1640, DMEM, X-VIVO 15^{®}, and the like. The tissue culture media can be supplemented with serum, amino acids, vitamins, cytokines, such as GM-CSF and/or IL-4, divalent cations, and the like, to promote differentiation of the cells. The dendritic cell precursors can be cultured in the serum-free media. Such culture conditions can optionally exclude any animal-derived products. A typical cytokine combination in a typical dendritic cell culture medium is about 500 units/ml each of GM-CSF (50 ng/ml) and IL-4 (10 ng/ml). Dendritic cell precursors, when differentiated to form immature dendritic cells, are phenotypically similar to skin Langerhans cells. Immature dendritic cells typically are CD14- and CD11c+, express low levels of CD86 and CD83, and are able to capture soluble antigens via specialized endocytosis. The immature DC expressed very high levels of CD86. Also, the population was mixed in terms of CD14 and CD11C. Though the majority were CD11c+, there were distinct subpopulations that were CD11c- and CD 14+.

The immature dendritic cells are matured to form mature dendritic cells. Mature DC lose the ability to take up antigen and display up-regulated expression of costimulatory cell surface molecules and various cytokines. Specifically, mature DC express higher levels of MHC class I and II antigens than immature dendritic cells, and mature dendritic cells are generally identified as being CD80+, CD83+, CD86+, and CD14-. Greater MHC expression leads to an increase in antigen density on the DC surface, while up regulation of costimulatory molecules CD80 and CD86 strengthens the T cell activation signal through the counterparts of the costimulatory molecules, such as CD28 on the T cells.

Mature dendritic cells of the present invention can be prepared (*i.e.,* matured) by contacting the immature dendritic cells with effective amounts or concentrations of a nucleic acid composition and a tumor antigen composition. Effective amounts of nucleic acid composition typically range from at most, at least, or about 0.01, 0.1, 1, 5, 10, to 10, 15, 20, 50, 100 ng or mg of nucleic acid per culture dish or per cell, including all values and ranges there between. Effective amounts of tumor antigen composition typically range from at most, at least, or about 0.01, 0.1, 1, 5, 10, to 10, 15, 20, 50, 100 ng or mg of protein per culture dish or per cell. In certain aspects 0.001 ng of tumor antigen/cell to 1 µg of tumor antigen/million cells) can be used. The tumor antigen composition can optionally be heat inactivated or treated (*e*.*g*., exposed to protease) prior to contact with dendritic cells. Maturing the immature dendritic cells with a nucleic acid composition and a tumor antigen composition primes the mature dendritic cells for a type 1 (Th-1) response.

The immature DC are typically contacted with effective amounts of a nucleic acid composition and a tumor antigen composition for at most, at least, or about 1, 2, 3, 4, 5, 6, 7, 8, 9, 10, 11, 12, to 10, 11, 12, 13, 14, 15, 16, 17, 18, 19, 20, 21, 22, 23, or 24 minutes, hours, or days. The immature dendritic cells can be cultured and matured in suitable maturation culture conditions. Suitable tissue culture media include AIM-V^{®}, RPMI 1640, DMEM, X-VIVO 15^{®}, and the like. The tissue culture media can be supplemented with amino acids, vitamins, cytokines, such as GM-CSF and/or IL-4, divalent cations, and the like, to promote maturation of the cells.

Maturation of dendritic cells can be monitored by methods known in the art. Cell surface markers can be detected in assays familiar to the art, such as flow cytometry, immunohistochemistry, and the like. The cells can also be monitored for cytokine production (*e.g*., by ELISA, FACS, or other immune assay). Dendritic cell precursors, immature dendritic cells, and mature dendritic cells, either primed or unprimed, with antigens can be cryopreserved for use at a later date. Methods for cryopreservation are well-known in the art. For example, U.S. Pat. No. 5,788,963.

### A. Genetically Modified Dendritic Cells

Certain aspects of the embodiments concern dendritic cells that have been genetically modified. In some aspects, the genetic modification comprises introduction of an exogenous transgene in the cells, such as an inhibitory nucleic acid. In further aspects, the transgene may be a suicide gene, such as a gene encoding thymidine kinase, under the control of an inducible promoter. Thus, in some aspects, after stimulating an immune response, administered dendritic cells can be killed-off by induction of the promoter controlling expression of the suicide gene.

In further aspects, the genetic modification comprises a genomic deletion or insertion in the cell population. For example, one or more HLA gene may be disrupted to render the dendritic cells as an effective HLA match for a subject to be treated.

Further aspects of the embodiments concern dendritic cells that have been genetically modified, such as to reduce the expression of CTLA-4. In some aspects, the genetic modification comprises introduction of an exogenous inhibitory nucleic acid specific to CTLA-4. In certain aspects, the inhibitory nucleic acid is a RNA, such as a RNA that is expressed from a DNA vector in the dendritic cells. In further aspects, the inhibitory nucleic acid may be a siRNAs, dsRNA, miRNA or shRNA that is introduced in the dendritic cells. A detailed disclosure of such RNAs is provided above.

In further aspects, the genetic modification comprises a genomic deletion or insertion in the cell population that reduces CTLA-4. In other aspects, the dendritic cells comprises a hemizygous or homozygous deletion within the CTLA-4 gene. For example, in some aspects, one or both copies of the CTLA-4 gene of a dendritic cell can be completely or partially deleted, such that expression the CTLA-4 polypeptideis inhibited. In some aspects, modification the cells so that they do not express one or more CTLA-4 gene may comprise introducing into the cells an artificial nuclease that specifically targets the CTLA-4 locus. In various aspects, the artificial nuclease may be a zinc finger nuclease, TALEN, or CRISPR/Cas9. In various aspects, introducing into the cells an artificial nuclease may comprise introducing mRNA encoding the artificial nuclease into the cells.

### III. Combination therapies

In order to increase the effectiveness of dendritic cell therapies of the embodiments, it may be desirable to combine these compositions with other agents effective in the treatment of the disease of interest.

The dendritic cell therapies may be administered in conjunction with molecules such as TLR agonists, Type I interferon (INF), AIMp1, a retinoic acid inducible gene-1 (RIG-1)-like receptor ligand or a cytosolic DNA (CDS) receptor ligand. The TLR agonist may be a TLR3, TLR7, TLR8 or TLR9 agonist. The Type I INF may be INF-α, IFN-β, IFN-ε, IFN-κ or IFN-ω. For example, the TLR-7 agonist may be selected from the group consisting of CL075, CL097, CL264, CL307, GS-9620, Poly(dT), imiquimod, gardiquimod, resiquimod (R848), loxoribine, and a ssRNA oligonucleotide. Exemplary TLR-9 agonists include a CpG oligodeoxynucleotide (CpG ODN). Other TLR agonists are described for example in U.S. Patent Publication No. 2014/0005255.

A RIG-I-like receptor (RLR) ligand, which are known in the art, refers to activator of RIG-I, Mda5, as well as LGP2 signaling. These ligands include, but are not restricted to, single-stranded RNA, double-stranded RNA, and 5'-triphosphate RNA. RIG-I-like receptor ligand also refers to any modification introduced in an RNA molecule that can lead to binding and activation of RIG-I, Mda5, and LGP2 leading to RLR-like biological activity. In some aspects, the RLR ligand may be a modulator of common adaptor protein such as IPS-1, also known as MAVS, VISA or CARDIF. For example, the RIG-1-like receptor ligand is selected from the group consisting of a MDA5 ligand, a LGP2 ligand, a ssRNA, a dsRNA, 5'ppp-dsRNA, Poly(dA:dT), and Poly(I:C).

The primed dendritic cell population may be administered in conjunction with a CDS receptor ligand. The CDS receptor ligand may be further defined as a cGAS-STING ligand. For example, the cGAS-STING ligand is bacterial cyclic-dinucleotides (CDNs). Other cGAS-STING agonists such as nucleic acid, a protein, a peptide, or a small molecule are described for example in International Patent Publication No. WO2015/077354.

As a non-limiting example, the treatment of cancer may be implemented with a primed dendritic cell composition of the present embodiments along with other anti-cancer agents. An "anti-cancer" agent is capable of negatively affecting cancer in a subject, for example, by killing cancer cells, inducing apoptosis in cancer cells, reducing the growth rate of cancer cells, reducing the incidence or number of metastases, reducing tumor size, inhibiting tumor growth, reducing the blood supply to a tumor or cancer cells, promoting an immune response against cancer cells or a tumor, preventing or inhibiting the progression of cancer, or increasing the lifespan of a subject with cancer. More generally, these other compositions would be provided in a combined amount effective to kill or inhibit proliferation of the cell. This process may involve contacting the cells with the anti-cancer peptide or nanoparticle complex and the agent(s) or multiple factor(s) at the same time. This may be achieved by contacting the cell with a single composition or pharmacological formulation that includes both agents, or by contacting the cell with two distinct compositions or formulations, at the same time, wherein one composition includes the dendritic cell composition and the other includes the second agent(s).

Treatment with the a dendritic cell composition may precede or follow the other agent treatment by intervals ranging from minutes to weeks. In embodiments where the other agent and dendritic cell composition are applied separately to the subject, one would generally ensure that a significant period of time did not expire between the time of each delivery, such that the agent and the dendritic cell composition would still be able to exert an advantageously combined effect on the cell. In such instances, it is contemplated that one may contact the cell with both modalities within about 12-24 hours of each other and, more preferably, within about 6-12 hours of each other. In some situations, it may be desirable to extend the time period for treatment significantly where several days (*e.g.,* 2, 3, 4, 5, 6 or 7 days) to several weeks (*e*.*g*., 1, 2, 3, 4, 5, 6, 7 or 8 weeks) lapse between the respective administrations.

Various combinations may be employed, where dendritic cell therapy is "A" and the secondary agent, such as radiotherapy, chemotherapy or anti-inflammatory agent, is "B":

| | | | | | | | |
|---|---|---|---|---|---|---|---|
| A/B/A | B/A/B | B/B/A | A/A/B | A/B/B | B/A/A | A/B/B/B | B/A/B/B |
| B/B/B/A | B/B/A/B | | A/A/B/B | A/B/A/B | | A/B/B/A | B/B/A/A |
| B/A/B/A | B/A/A/B | | A/A/A/B | B/A/A/A | | A/B/A/A | A/A/B/A |

In certain embodiments, administration of dendritic cell therapy of the present embodiments to a patient will follow general protocols for the administration of chemotherapeutics, taking into account the toxicity, if any, of the vector. It is expected that the treatment cycles would be repeated as necessary. It also is contemplated that various standard therapies, as well as surgical intervention, may be applied in combination with the described hyperproliferative cell therapy.

### A. Chemotherapy

Cancer therapies also include a variety of combination therapies. A dendritic cell composition may be administered (or formulated) in conjunction with a chemotherapeutic agent. For example, in some aspects the chemotherapeutic agent is a protein kinase inhibitor such as a EGFR, VEGFR, AKT, Erb1, Erb2, ErbB, Syk, Bcr-Abl, JAK, Src, GSK-3, PI3K, Ras, Raf, MAPK, MAPKK, mTOR, c-Kit, eph receptor or BRAF inhibitors. Nonlimiting examples of protein kinase inhibitors include Afatinib, Axitinib, Bevacizumab, Bosutinib, Cetuximab, Crizotinib, Dasatinib, Erlotinib, Fostamatinib, Gefitinib, Imatinib, Lapatinib, Lenvatinib, Mubritinib, Nilotinib, Panitumumab, Pazopanib, Pegaptanib, Ranibizumab, Ruxolitinib, Saracatinib, Sorafenib, Sunitinib, Trastuzumab, Vandetanib, AP23451, Vemurafenib, MK-2206, GSK690693, A-443654, VQD-002, Miltefosine, Perifosine, CAL101, PX-866, LY294002, rapamycin, temsirolimus, everolimus, ridaforolimus, Alvocidib, Genistein, Selumetinib, AZD-6244, Vatalanib, P1446A-05, AG-024322, ZD1839, P276-00, GW572016 or a mixture thereof.

Yet further combination chemotherapies include, for example, alkylating agents such as thiotepa and cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (*e*.*g*., calicheamicin, especially calicheamicin gammalI and calicheamicin omegaI1; dynemicin, including dynemicin A; bisphosphonates, such as clodronate; an esperamicin; as well as neocarzinostatin chromophore and related chromoprotein enediyne antiobiotic chromophores, aclacinomysins, actinomycin, authrarnycin, azaserine, bleomycins, cactinomycin, carabicin, carminomycin, carzinophilin, chromomycinis, dactinomycin, daunorubicin, detorubicin, 6-diazo-5-oxo-L-norleucine, doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalarnycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elformithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2-ethylhydrazide; procarbazine; PSK polysaccharide complex; razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; taxoids, *e*.*g*., paclitaxel and docetaxel gemcitabine; 6-thioguanine; mercaptopurine; platinum coordination complexes such as cisplatin, oxaliplatin and carboplatin; vinblastine; platinum; etoposide (VP-16); ifosfamide; mitoxantrone; vincristine; vinorelbine; novantrone; teniposide; edatrexate; daunomycin; aminopterin; xeloda; ibandronate; irinotecan (*e*.*g*., CPT-11); topoisomerase inhibitor RFS 2000; difluorometlhylornithine (DMFO); retinoids such as retinoic acid; capecitabine; carboplatin, procarbazine, plicomycin, gemcitabien, navelbine, farnesyl-protein tansferase inhibitors, transplatinum, and pharmaceutically acceptable salts, acids or derivatives of any of the aboveThe compositions provided herein may be used in combination with gefitinib. The present embodiments may be practiced in combination with Gleevac (e.g., from about 400 to about 800 mg/day of Gleevac may be administered to a patient). In certain embodiments, one or more chemotherapeutic may be used in combination with the compositions provided herein.

### B. Radiotherapy

Other factors that cause DNA damage and have been used extensively include what are commonly known as γ-rays, X-rays, and/or the directed delivery of radioisotopes to tumor cells. Other forms of DNA damaging factors are also contemplated such as microwaves and UV-irradiation. It is most likely that all of these factors effect a broad range of damage on DNA, on the precursors of DNA, on the replication and repair of DNA, and on the assembly and maintenance of chromosomes. Dosage ranges for X-rays range from daily doses of 50 to 200 roentgens for prolonged periods of time (3 to 4 wk), to single doses of 2000 to 6000 roentgens. Dosage ranges for radioisotopes vary widely, and depend on the half-life of the isotope, the strength and type of radiation emitted, and the uptake by the neoplastic cells.

The terms "contacted" and "exposed," when applied to a cell, are used herein to describe the process by which a therapeutic composition and a chemotherapeutic or radiotherapeutic agent are delivered to a target cell or are placed in direct juxtaposition with the target cell. To achieve cell killing or stasis, both agents are delivered to a cell in a combined amount effective to kill the cell or prevent it from dividing.

### C. Gene Therapy

The secondary treatment may be a gene therapy in which a therapeutic polynucleotide is administered before, after, or at the same time as the therapeutic composition. Viral vectors for the expression of a gene product are well known in the art, and include such eukaryotic expression systems as adenoviruses, adeno-associated viruses, retroviruses, herpesviruses, lentiviruses, poxviruses including vaccinia viruses, and papiloma viruses, including SV40. Alternatively, the administration of expression constructs can be accomplished with lipid based vectors such as liposomes or DOTAP:cholesterol vesicles. All of these method are well known in the art (see, *e.g.* Sambrook *et al.,* 1989; Ausubel *et al.,* 1998; Ausubel, 1996).

### D. Surgery

Approximately 60% of persons with cancer will undergo surgery of some type, which includes preventative, diagnostic or staging, curative and palliative surgery. Curative surgery is a cancer treatment that may be used in conjunction with other therapies, such as the treatments provided herein, chemotherapy, radiotherapy, hormonal therapy, gene therapy, immunotherapy and/or alternative therapies.

Curative surgery includes resection in which all or part of cancerous tissue is physically removed, excised, and/or destroyed. Tumor resection refers to physical removal of at least part of a tumor. In addition to tumor resection, treatment by surgery includes laser surgery, cryosurgery, electrosurgery, and miscopically controlled surgery (Mohs' surgery). It is further contemplated that the present embodiments may be used in conjunction with removal of superficial cancers, precancers, or incidental amounts of normal tissue. In some aspects, following tumor resection a dendritic cell composition of the embodiments is administered to lymphoid tissue that drained the previous site for the tumor.

### IV. Examples

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice..

### Example 1 - Materials and Methods

As used in the examples described below, Eight-to-twelve-week-old C57BL/6, Balb/c, and FVB mice were obtained from Harlan Laboratories (Indianapolis, IN) or from the Jackson Laboratory (Barr Harbor, ME). H2-DM knockout mice in the C57BL/6 background were a kind gift from Dr. Jenny Ting at the University of North Carolina, Chapel Hill. Pro-Cat/JOCK1 transgenic mice in the FVB background were created in the laboratory of Dr. David Spencer at Baylor College of Medicine, Houston TX as described (Carstens *et al.,* 2014). All mice were maintained in accordance with the specific IACUC requirements of Baylor College of Medicine.

*Preparation of Vaccine Materials, Loading of DC, and In vitro CoCulture-*Seminal vesicle (SV) and prostate were harvested from 10 week old male mice and immediately frozen at -80° C. RAW264.7, B16-F10, 4T1, and WPMY-1 cell lines were obtained from the American Type Culture Collection (ATCC, Manassas, VA), grown to confluence in T225 flasks (Corning Lifesciences, Tewksbury, MA) at 37°C, 5% CO₂, harvested, and also immediately frozen at -80° C. Peptides were reconstituted in an 80:20 dH₂O:DMSO solution at 10mg/ml and stored at -80°C. To generate MHC class I (mRNA) or II (lysate) determinants, tissue fractions were first disrupted using a Polytron PT1200E tissue homogenizer (Kinematica, Inc, Bohemia, NY). To generate cell lysates, homogenized tissue suspensions were diluted to 50 mg/ml in PBS (Life Technologies, Carlsbad, CA), subjected to repetitive freeze-thaw cycles, and stored at -20° C. To generate mRNA, total RNA was isolated from homogenized tissue using Trizol reagent (Life Technologies, Carlsbad, CA) according to the manufacturer's instructions, and mRNA was isolated from total RNA using an Oligotex mRNA Maxi Kit (Qiagen, Valencia, CA) also according to the manufacturer's instructions. mRNA was quantitated with a Nanodrop spectrophotometer (Thermo Scientific) and integrity was verified by gel electrophoresis. Human (Decker *et al.,* 2006; Decker *et al.,* 2009) and wild type mouse (Konduri *et al.,* 2013) dendritic cells were prepared, loaded, and matured as described. Use of siRNA was performed according to the manufacturer's instructions (Thermo Scientific - Dharmacon). Maturation cocktail of H2-DM^{-/-} DC was additionally supplemented with 1 µg/ml CpG-ODN (InvivoGen). *In vitro* co-cultures were performed as described previously (Decker *et al.,* 2006; Decker *et al.,* 2009; Konduri *et al.,* 2013).

*Vaccination* - All mice vaccinated therapeutically were administered 5 x 10⁴-5 x 10⁵ DC i.p. and 0.5 mg imiquimod (LC Labs, Woburn, MA) suspended but not solubilized in 20% DMSO/80% AIM-V, also i.p. Mice were vaccinated one to four times as shown in FIG. 12. Different vaccine treatments included DC loaded with mRNA, DC loaded with lysate, DC concurrently loaded with either homologous or heterologous mRNA and lysate, DC loaded with homologous mRNA and lysate and AIMp1 siRNA, and unloaded DC. Multiple vaccinations were given 10 days apart. Mice vaccinated with SIINFEKL (SEQ ID NO: 1)/Ova loaded DC were vaccinated in the footpad and did not receive imiquimod.

*Histological and Gross Analysis -* Paraffin sections were stained with hematoxylin and eosin for gross histological analysis by light microscopy using an Olympus CX41 microscope (Olympus corporation, Center Valley, PA) with an Olympus DP70 digital camera (Olympus Corporation). Blinded histopathological scoring of prostate cancer consisted of a four-point scale based upon predominant stage of disease present among all anterior, ventral, and dorsolateral fields observed in two sections of differing depths: 0 = normal, 1 = hyperplasia, 2 = PIN, 3 = adenocarcinoma, 4 = transitional. Half points were permitted if a predominant stage could not be discerned.

*MRI Analysis* - MRI of the prostate and seminal vesicles was performed using a 9.4T, 21 cm bore horizontal scanner with a 35 mm volume resonator (Bruker BioSpin, Billerica, MA). The imaging parameters used to obtain three dimensional (3D) Turbo rapid acquisition with relaxation enhancement (RARE) images were as follows: TR = 3000 ms; Effective TE = 30 ms; FOV = 30 mm³; matrix = 128 x 128 x 128; RARE Factor = 8; Number of Averages = 1. Images were obtained using Paravision software version 5 (Bruker BioSpin). During imaging, mice were anesthetized with 0.25% isoflurane (Abbott, Abbott Park, IL) mixed with oxygen and the core temperature was maintained at 37° C. MRI images were analyzed using Amira 3.1 software (Visage Imaging, San Diego, CA).

*CTLA-4*/*sCTLA-4 RT-PCR Assay -* Loaded, matured DC were resuspended in 1mL Trizol (Life Technologies) at < 1 x 10⁷ cells per sample and total RNA was extracted according to manufacture's instructions. RNA was treated with 1µg/µl DNase I (Invitrogen). cDNA was synthesized from the DNase-treated RNA sample using the SuperScript^{™} III First-Strand Synthesis kit (Life Technologies) and amplified by PCR for 35 cycles at an annealing temperature of 55° C with CTLA-4 Fwd primer: ATGGCTTGCCTTGGATTTCAGCGGC (SEQ ID NO: 12) and CTLA-4 Rev primer: TCAATTGATGGGAATAAAATAAGGCTG (SEQ ID NO: 13). Primers were designed to amplify transcripts corresponding to both soluble and membrane-bound CTLA-4 isoforms.

*Quantitation of Western Blot Images -* Western blot chemiluminescent signal was detected using a ChemiDoc XRS digital imaging system running Image Lab software Version 2.0.1 (Bio-Rad Laboratories, Hercules, CA). All Western blots were quantitated by densitometry of Ponceau S (Sigma-Aldrich) stained membranes. Contamination of supernatants with residual cell lysate or debris from cell death was controlled for by immunostaining with anti-β-actin (Santa Cruz) and additional densitometry. Densitometry was performed using ImageJ software (NIH; Bethesda, MD). For detection of both sCTLA-4 and AIMp1 on a single membrane, the membrane was typically probed first with anti-CTLA-4 after which it was stripped in Western Blot Restore buffer (Pierce, Rockford, IL) according to the manufacturer's instructions and re-probed with anti-AIMp1.

*Pro-Cat*/*JOCK1 Prostate Cancer Treatment Model* - Pro-Cat/JOCK1 mice were housed in a pathogen-free facility following approved IACUC protocols. Double transgenic mice were generated and genotyped as described (Carstens *et al.,* 2014). Mice were treated biweekly starting at six weeks of age by i.p. injections of 100 µl AP20187 (Ariad Pharmaceuticals) at 2 mg/kg in drug diluent (16.7% propanediol, 22.5% PEG400, 1.25% TWEEN 80). Mice were vaccinated i.p. in the lower urogenital region after 24 weeks of AP20187 treatment with 5 x 10⁴ - 4 x 10⁵ loaded DC + 0.5 mg particulate imiquimod (LC LABS) in 100 µl 20% DMSO or injected with 0.5 mg imiquimod only. Mice received four vaccine + imiquimod injections spaced at 10 day intervals. AP20187 injections were maintained biweekly until sacrifice.

*Spontaneous Canine Oligodendroglioma Treatment Model -* Upon diagnosis of CNS malignancy by clinical MR imaging, large (> 25 kg) canine patients were enrolled in a non-randomized phase I trial following informed consent of the owners under an IACUC protocol established through the Translational Genomics Research Institute. Canine patients underwent craniotomy and conservative tumor resection after which the excised tumor was flash frozen in liquid nitrogen. To prepare vaccine antigens, the thawed tumor specimen was sub-divided into soluble lysate and mRNA components, and antigenic fractions were prepared as described above. Subsequently, patients were mobilized with G-CSF (Neupogen, Amgen, Thousand Oaks, CA) and peripheral blood mononuclear cells (PBMC) were harvested. Canine DCs were generated from the adherent monocytic fraction by six days of culture in AIM-V medium supplemented with 10% canine serum (Equitech Bio), 1% anti-anti (Life Technologies), 30 ng/ml rcGM-CSF and 10 ng/ml rcIL-4 (both from R&D Systems). Following loading with tumor antigens as described above, loaded DC matured using the same culture medium as described but supplemented additionally with 10 ng/ml rcIL-1β, 15 ng/ml rcIL-6, 10 ng/ml rcTNF-α (all from R&D Systems) and 1 µg/ml PGE₂ (Sigma-Aldrich). DC were then harvested and resuspended in 2 x 500 µl aliquots PBS for bilateral injection into the vicinity of the deep cervical lymph nodes by means of ultrasound sonography. If multiple doses were administered, injections were spaced two weeks apart. During the course of treatment, animals were adjuvanted with 12 weeks of human IFN-α administered subcutaneously thrice weekly at two to eight million units per dose. Tumor volume was determined from digital MRI measurements by the following formula: Volume = 4/3π(smallest radius)² x (largest radius/smallest radius).

*Statistical Analysis -* Statistical significance was defined as p < 0.05 (* = p < 0.05, ** = p < 0.01) and was determined by Student's unpaired or paired t-test with one or two tails as statistically appropriate. Statistical differences between multiple groups were validated by one-way or two-way ANOVA. Statistical tests were performed with Microsoft Excel 2008 for the Macintosh Version 12.0. All normalized quantitation graphs were derived from three independent experiments unless stated otherwise and with error bars = +/- SD.

*Reagents* - Antibodies: αHuman CTLA-4 (ELISA) (eBioscience, San Diego, CA); αHuman/mouse CTLA-4 (WB) (Abcam; Cambridge, MA); αHuman/mouse AIMP1 (Lifespan Biosciences Inc, Seattle, WA); αMouse IL-12p70 (ELISA) (BD Biosciences, San Jose, CA); αHuman CD8 (ICH) (Biorbyt; San Francisco, CA), αMouse CD8 (flow cytometry), αMouse CD25, αMouse CD3, and αMouse CD4 (BD Biosciences); αMouse CD8 (*in vivo* depletion) and isotype control (BioXCell, West Lebanon, NH). αHuman/mouse β-actin was purchased from Santa Cruz Biotechnologies (Santa Cruz, CA). αHLA-A,B,C was purchased from BioLegend, San Diego, CA. HLA typing antibodies: αHLA-A2-FITC (BD Biosciences), αHLA-B8-biotin (Abcam), and unconjugated αHLA-DR3/DR6 (Lifespan Biosciences). TLR agonists: TLR-3 agonist poly(I:C)-rhodamine, TLR-9 agonist CpG ODN-FITC, and TLR-5 agonist flagellin were obtained from InvivoGen (San Diego, CA). TLR-4 agonist LPS was obtained from Sigma-Aldrich (St. Louis, MO). DC uptake of poly(I:C)-rhodamine and CpG-FITC was confirmed and quantitated by fluorescent microscopy and flow cytometry using an LSR II flow cytometer (BD Biosciences) and analyzed with FlowJo version 10.0.00003 for the MacIntosh (Tree Star Inc, Ashland, OR). All TLR agonists were used at a concentration of 1 µg/ml. Peptides: Influenza A New Caledonia hemagglutinin peptides WLTGKNGL (SEQ ID NO: 3), RNLLWLTGKNGLYPN (SEQ ID NO: 2), VLLENERTL (SEQ ID NO: 5), and ELLVLLENERTLDFH (SEQ ID NO: 4) (described previously in Decker *et al.,* 2009) as well as methionine-for-glycine substituted derivatives WLTMKNML (SEQ ID NO: 8) and RNLLWLTMKNMLYPN (SEQ ID NO: 9) were synthesized by United BioSystems (Herndon,VA). Ovalbumin H-2K^{b} immunodominant peptide SIINFEKL (SEQ ID NO: 1) was synthesized by Anaspec (Freemont, CA). H-2D^{b} CLIP-overlapping MRMATPLLM (SEQ ID NO: 6) was synthesized by United Biosystems. Recombinant ovalbumin protein was purchased from InvivoGen. Recombinant eGFP protein was purchased from Biovision (Moutainview, CA). Other: AIMp1 (SCYE1, mouse and human), β2-microglobulin (mouse and human), and HLA-DM (human) siGenome SMART Pools and non-targeting siRNA pools were purchased from Thermo Scientific (Wilmington DE). Purified GFP mRNA was purchased from Stemgent (Cambridge, MA).

*Co-Immnunoprecipitation Assay* - DC were loaded as indicated and matured for two days before lysis with 1% NP-40 buffer + protease cocktail inhibitor (both from Sigma-Aldrich). Debris was pelleted at 14,000 rpm for 20 minutes at 4° C in a tabletop microfuge, and subsequent cell lysates were precleared with Protein G plus-Agarose Bead suspension IP04 (EMD Millipore; Darmstadt, Germany) for 1 hour at 4° C. Lysates were then rotated overnight at 4° C with Protein G plus beads coated with anti-AIMp1 (Lifespan Biosciences) or anti-HLA-A,B,C (BioLegend). Beads were then washed three times in 1% NP-40 buffer, twice in PBS, and immunoprecipitate was collected by boiling in 2% SDS (Sigma-Aldrich) denaturing buffer prior to analysis by PAGE.

*⁵¹Cr Lysis Assay -* ⁵¹Cr Lysis Assay was performed as described previously (Decker *et al.,* 2006).

*4T1-luc2 Tumor Model -* 4T1-luc2 tumor cells were prepared by cloning of the PCR-amplified luc2 cassette from pGL4.10 into the pCDH-CMV-MCS-EF1-Hygro expression vector. Linearized vector was electroporated into 4T1 parental cells, and 100 µg/ml hygromycin selection was applied for two weeks. To generate tumors, Balb/c mice were inoculated intradermally with 2.5 x 10⁵ 4T1-luc2 cells. Tumor growth was monitored by caliper measurement and IVIS every other day. IVIs images were acquired following i.p. injection of 0.5 mg d-luficerin (Regis Technologies, Morton Grove, IL) in 100 µl dilulent.

### Example 2 - Characterization of AIMpl release and T_{H}1-polarization of mouse DC

Previous work implied the existence of a TLR- and IFN-independent mechanism of DC T_{H}1 polarization initiated by loading with antigenically homologous MHC class I and II determinants (Decker *et al.,* 2009). To determine whether AIMp1 played a role in this process, the inventors loaded DC with homologous class I and II antigenic determinants and assayed for T_{H}1 polarization and AIMp1 release. Mouse DC secreted 10-fold more IL-12p70 **(****FIG. 1A****)** as well as significantly more AIMp1 when homologously loaded with cell line or primary tissue determinants (mRNA and lysate) in comparison to singly- or heterologously-loaded DC **(****FIG. 1B****).** In addition to augmented AIMp1 release, T_{H}1 DC secreted significantly *less* sCTLA-4 when class I and II determinants were homologous **(****FIG. 1C****).** In agreement with studies indicating a role for AIMp1 in T_{H}1 polarization, AIMp1 siRNA knockdown dramatically reduced IL-12 secretion in response to homologous loading **(****FIG. 1A****)** and restored sCTLA-4 secretion **(****FIG. 1D****).** Accordingly, homologously-loaded DC generated significantly more activated CD8⁺ T-cells *in vitro* than either heterologously-loaded DC or homologously-loaded DC electroporated with AIMp1 siRNA **(****FIG. 1E****).** The inventors next used the H-2K^{b} class I ovalbumin (Ova) epitope SIINFEKL and whole Ova protein as a source of homologous class II antigen to explore this same phenomenon *in vivo.* As with *in vitro* experiments, expansion of CD3⁺, CD3⁺CD8⁺, and especially CD3⁺CD8⁺CD25⁺ populations were observed only in DC electroporated with SIINFEKL peptide and simultaneously loaded with Ova. In contrast, singly-loaded DC, heterologously-loaded DC, or homologously-loaded DC treated with AIMp1 siRNA exhibited no differences in relevant T-cell populations **(****FIG. 1F****).** Taken together, the data indicate that loading of DC MHC class I and II with antigenically-related determinants promotes T_{H}1-skewing which includes an increase in the secreted AIMp1/sCTLA-4 ratio, downstream release of IL-12, and the enhanced generation of activated CD8⁺ T-cells.

### Example 3 - Human system DC AIMp1/sCTLA-4 release

When human DC were homologously-loaded with the model GFP antigen (GFP mRNA and recombinant GFP protein), a significant increase in the AIMp1/sCTLA-4 ratio was observed **(****FIGS. 2A-B).** The inventors then transitioned from multi-epitopic systems to previously characterized MHC binding peptides (Decker *et al.,* 2009). When class I and II peptides overlapped in amino acid sequence, a decrease in sCTLA-4 secretion and an increase in AIMp1 secretion were routinely observed from loaded DC **(****FIGS. 2C-E).** The differential in sCTLA-4 secretion was not observed pre-maturation, yet AIMp1 release from DC loaded with overlapping peptides began almost immediately and prior to maturation **(****FIG. 8****),** suggesting an early upstream role for AIMp1 release. Since all peptides were prepared identically, had no effect upon AIMp1 and sCTLA-4 secretion when added singly or in a heterologous fashion, and possess no known PRR ligands, it was thought that the mechanism of AIMp1 secretion/sCTLA-4 ablation was indeed triggered by concurrent homologous MHC loading and not by PRR agonism. Nonetheless, these experiments were repeated in the presence of various TLR agonists. Neither poly(I:C), LPS, flagellin, nor CpG-ODN altered sCTLA-4 or AIMp1 secretion from mouse (not shown) or human DC **(****FIGS. 2D-E)** in the absence of antigenic loading. Additionally, use of various homologous class I and II binding peptides ablated both CTLA-4 and its corresponding mRNA when concurrently added to the DC, though the addition of single or heterologous class I and II binding peptides had no such effect **(****FIG. 2F****).** AIMp1 showed no differences at the mRNA level irrespective of antigenic load.

To verify the importance of MHC binding to the function of this unique T_{H} polarization cue, the inventors utilized H2-DM^{-/-} DC. The H2-DM molecular chaperone is responsible for removing the CLIP peptide of the invariant chain from the MHC class II binding pocket. In the absence of H2-DM, CLIP is bound almost irreversibly to the class II binding pocket in the I-A^{b} haplotype, thereby abrogating the ability to load exogenous antigen (Martin *et al.,* 1996; Miyazaki *et al.,* 1996). The inability to load exogenous antigen is the primary molecular deficit of H2-DM^{-/-} DC. H2-DM^{-/-} DC have no known defects in TLRs, NLRs, or other PRRs and have previously been shown to respond appropriately to TLR agonism (Strong *et al.,* 1997). Very interestingly, H2-DM^{-/-} DC have also been reported to display a T_{H}2 polarized phenotype physically dependent upon the presence of bound CLIP peptide (Rohn *et al.,* 2004). When loaded with homologous mRNA and lysate, H2-DM^{-/-} DC displayed no differential secretion of AIMp1 or sCTLA-4 nor stimulated enhanced generation of activated CD8⁺ T-cells *in vivo* when loaded with SIINFEKL (SEQ ID NO: 1) and Ova **(****FIGS. 3A-B** **and** **FIG. 1F****).** Rather, H2-DM^{-/-} DC constitutively and invariantly secreted low levels of AIMp1 and high levels of sCTLA-4 **(****FIGS. 3A-B).** DC AIMp1/sCTLA-4 release and downstream T_{H}1 responses were regulated via sequence homology of MHC-bound peptides.

Because H2-DM^{-/-} DC permanently maintain CLIP within the MHC Class II binding groove, the only theoretical manner by which to stimulate T_{H}1 polarization in H2-DM^{-/-} DC by the cue described herein would be via loading of a class I binding peptide with significant homology to CLIP, *i.e.* with amino acid sequence overlapping the MHC class II bound-CLIP sequence LPKSAKPVSQMRMATPLLMRPMSM (SEQ ID NO: 14) (Ghosh *et al.,* 1995). To test this hypothesis, the inventors designed a class I peptide predicted to bind H-2D^{b} and possessing full sequence overlap with CLIP (MRMATPLLM, SEQ ID NO: 6). The inventors then loaded H2-DM^{-/-} DC with either this CLIP-specific H-2D^{b} class I peptide or the well-established H-2^{b} class I SIINFEKL (SEQ ID NO: 1) peptide as a control. Substantial AIMp1 release was observed in a dose-dependent fashion only from the cells loaded with H-2D^{b} CLIP **(****FIG. 3C****).** Similarly, diminution of sCTLA-4 secretion was only observed from H2-DM^{-/-} DC loaded with H-2D^{b} CLIP, again in a dose-dependent fashion. Subsequent coculture of loaded H2-DM^{-/-} DC with wild type, syngeneic splenocytes led to an increase in CD8⁺CD25⁺ T cells only when H2-DM^{-/-} DC were loaded with H-2D^{b} CLIP **(****FIG. 3D****).** These data validate previous results and suggest high level specificity of an intrinsic DC mechanistic process that compares the amino acid sequences of peptides concurrently bound to MHC class I and II.

To determine the degree of class I and II sequence homology required for perturbation of AIMp1 and sCTLA-4 secretion, the inventors utilized two different pairs of homologous binding peptides that were identical save for the substitution of two non-anchor glycine residues with methionines **(****FIG. 3E****).** Loading of human DC with these peptide pairs indicated that substitution of one or the other HLA class I or II binding peptide was sufficient to prevent abrogation of CTLA-4 secretion and augmentation of AIMp1 secretion whereas the compensatory substitution on the reciprocal peptide was sufficient to restore the previously seen homologous phenotype **(****FIGS. 3E-F).** Tandem mass spectroscopy (not shown) validated by coIP and western blot indicated that AIMp1 interacts prominently with both MHC class I and II molecules, especially within matured DC **(****FIG. 9****).** Using homologous and heterologous peptide pairs, the inventors then demonstrated by AIMp1 coIP that AIMp1/MHC interaction was significantly decreased only when homologous class I and II peptides were loaded **(****FIG. 3G****),** an observation well-correlated with increased AIMp1 secretion from the cell. Similar findings were not observed when multiple class I, class II, or heterologous class I and II peptides were loaded. Experiments using mRNA and lysate generated identical results: AIMp1 remained bound to MHC in DC loaded singly or with heterologous mRNA and lysate whereas little AIMp1 remained bound to MHC in DC loaded with homologous mRNA and lysate **(****FIG. 10****).**

To further determine in the human system if T_{H}1 polarization resulting from sequence overlap between class I and II epitopes could occur independently from traditional MHC binding, the inventors utilized siRNA against either β2-microglobulin or HLA-DM, significantly impacting the ability of, respectively, MHC class I or MHC class II to be loaded with peptide antigen. When the ability to load either class I or class II was impeded, DC lost the ability to modulate AIMp1 and CTLA-4 secretion in response to homologous class I and II peptide loading **(****FIGS. 11A-B),** a finding consistent with previous data derived from the mouse **(****FIGS. 1F** **and** **3****).** In total, the data firmly support the hypothesis that DC possess a TLR-independent, antigenic sequence-dependent mechanism by which to modulate the release of important signaling molecules, the perturbations of which exert highly significant effects upon downstream T_{H} polarization and the development of the CD8⁺ T-cells (Decker *et al.,* 2006; Decker *et al.,* 2009).

### Example 4 - Physiologic Relevance of Sequence-dependent Mechanism

To ascertain physiologic relevance of this newly-characterized mechanism, the inventors characterized the ability of homologous antigenic loading to break tolerance to normal immunologic self. The inventors generated multiple homologously-loaded DC vaccines against wild type tissues including the seminal vesicle (SV) and prostate, intertwined but antigenically distinct urogenital organs easily visualized by MRI. Wild type mice were given 1 - 4 intraperitoneal (i.p) injections **(****FIG. 12****)** of 0.5 - 2.0 x 10⁵ DC loaded with SV mRNA and lysate along with *in situ* imiquimod (imq), an adjuvant that mimics the environment of viral infection by stimulation of plasmacytoid DC (pDC). Within one month of treatment, mice injected with SV-loaded DC displayed characteristic pathologic changes that included fibrosis and necrosis, smooth muscle and epithelial hyperplasia, luminal dilation and clumping, and a mixed lineage inflammatory infiltrate **(****FIG. 4A****).** SV eradication could be monitored longitudinally by MRI, with a compartmentalized loss of MR imageable tissue observed after one month of treatment **(****FIG. 4B****).** By six months post-vaccination, mice that received SV-loaded DC retained only vestigial structures comprised primarily of fibrotic tissue **(****FIG. 4C****).** CD8⁺ infiltration was confirmed by immunohistochemistry **(****FIG. 4D****).** To demonstrate vaccine specificity and memory, splenocytes were harvested from either control or SV-vaccinated mice at two months post-vaccination and adoptively transferred into naive mice without additional adjuvantation. **(****FIG. 13****).** Within six weeks, SV in adoptively-transferred mice displayed the same characteristic immunopathology as those that received primary vaccination **(****FIG. 4E****).**

The prostate is located adjacent to the SV, sharing borders along its anterior and lateral lobes. DC homologously loaded with wild type prostate mRNA and lysate were administered **(****FIG. 12****),** and mice were monitored by MRI. In comparison to control mice which maintained normal pathology **(****FIGS. 5A-C),** vaccine-treated mice displayed weaker prostate MRI signal **(****FIG. 5D****)** corresponding to a loss of tissue **(****FIG. 5E**) and demonstrated immunopathology similar to that of vaccinated SV **(****FIG. 5F****).** Fibrosis was not observed as a result of prostate vaccination, rather prostate tissue tended to disappear entirely **(****FIGS. 5G-****H).** The inventors additionally illustrated the ability to generate enhanced CD8⁺ responses against the human normal prostate cell line WPMY-1 by homologous-loading of partially-HLA-matched human DC with WPMY-1 antigenic determinants **(****FIG. 14****),** suggesting potential applicability to human therapy.

The generation of vaccine specificity against two antigenically and spatially-related self-tissue systems allowed discernment of immunologic specificity. As shown, when mice were treated with prostate-loaded DC, pathology was exclusively prostate-specific **(****FIG. 5I****).** Conversely, when mice were treated with SV-loaded DC, pathology was exclusively SV specific **(****FIG. 5J****).** Indeed, vaccination of 69 mice against immunologic self across a range of different doses did not generate observable off-target effects (not shown). Collectively, appropriate vaccine reactivity was observed in 59% of mice vaccinated with homologously-loaded DC (37/63) but 0% of mice vaccinated with tissue lysate-loaded DC (n=35, p<0.001) and 0% of mice vaccinated with tissue mRNA-loaded DC (n=12, p<0.001).

### Example 5 - Physiologic Neoplasia Study

To determine the ability of this strategy to address physiologic neoplasia, the inventors utilized a variety of different model systems. In the 4T1 breast cancer model, cohorts of mice with day 8 established 4T1 luc2-expressing tumors were given a single dose of homologous vaccine (derived from parental, luc2⁻ 4T1 cells) or adjuvant only. While 5 of 6 adjuvant-treated mice developed metastases and died, the vaccinated mice maintained relatively small tumors and demonstrated no obvious morbidity at the conclusion of the experiment **(****FIGS. 15A-B).** To demonstrate the dependence of tumor control upon CD8⁺ cells (and therefore T_{H}1 immunity) in this model, naive animals were given two doses of homologous vaccine then depleted of CD8⁺ cells. Splenocytes were then harvested and adoptively transferred into syngenic animals pre-inoculated with 2.5 x 10⁵ 4T1-luc2 tumor cells. As indicated **(****FIGS. 15C-D),** mice adoptively-transferred with isotype-depleted splenocytes exhibited a highly significant survival advantage over those adoptively-transferred with CD8-depleted splenocytes, indicating a critical role for CD8+ cells in control of both primary tumor control and metastatic spread. Interestingly, mice adoptively transferred with isotype-depleted splenocytes derived from heterologously-vaccinated (4T1 mRNA/B16.F10 lysate) animals fared worst of all.

To determine the effects of homologous vaccination in a model of high physiologic relevance, the inventors utilized the transgenic Pro-Cat/JOCK1 model in which physiologic autochthonous prostate cancer develops upon induced FGFR1 and β-catenin signaling in prostatic epithelium (Carstens *et al.,* 2014). In this model, mice progress from prostatic hyperplasia (8 weeks) through prostatic intraepithelial neoplasia (mPIN, 12 weeks), adenocarcinoma (24 weeks), and transitional sarcomatoid (60 weeks) stages. After inducing prostate adenocarcinoma for 24 weeks, mice were sacrificed and cancerous prostate was excised to generate adenocarcinoma stage antigenic preparations. Subsequent cohorts of mice were then induced for 24 weeks and vaccinated *therapeutically* with homologously-loaded vaccine. Vaccinated mice were sacrificed after an additional four months, and cancer progression was determined by blinded pathological scoring of H&E stained prostate. Mice receiving DC loaded with adenocarcinoma-stage antigens progressed through hyperplasia but largely arrested at mPIN, displaying relatively little histopathologic adenocarcinoma **(****FIGS. 6B and 6D****).** Additionally, vaccinated mice exhibited lymphocyte-infiltrated acini (*e.g.* **FIG. 6B****.****1** **inset**) not observed in unvaccinated, control-vaccinated, or adjuvant-only groups. Induced mice that received only adjuvant displayed typical adenocarcinoma **(****FIG. 6C****).** Uninduced control mice that received complete vaccine regimen indicated no cross-reactivity with normal prostate **(****FIG. 6A****).** Moreover, vaccination appeared to exhibit a dose-responsive effect **(****FIGS. 6E-J).**

Lastly, the inventors tested this approach on spontaneous brain tumors in a large animal system to demonstrate the feasibility and safety of this approach in a clinical veterinary setting. In brief, upon diagnosis of CNS malignancy by MRI, two large (> 25 kg) canine veterinary patients were recruited following informed consent of the owners. Instead of receiving standard of care palliative steroids, canines underwent craniotomy and conservative tumor resection. The tumor specimen was subdivided into soluble lysate and mRNA components. Subsequently, canine patients were mobilized with G-CSF (Neupogen) and peripheral blood mononuclear cells (PBMC) were harvested. The adherent monocytic fraction was differentiated into DC which were simultaneously loaded with tumor lysate and mRNA subfractions and matured. DC were then harvested and resuspended in PBS for injection into the vicinity of the deep cervical lymph nodes by means of ultrasound sonography. In conjunction with vaccination, animals were adjuvanted with 12 weeks of human IFN-α administered subcutaneously thrice weekly at two to eight million units per dose. In addition to demonstrating the safety and feasibility of this approach, each animal also exhibited rapid and significant tumor shrinkage at the outset of vaccination. The first animal received a single dose of 5 x 10⁵ vaccine cells and exhibited 50% tumor regression at one-month follow-up **(****FIGS. 7A-D).** The second animal received 5 x 10⁶ vaccine cells over the course of three administrations and exhibited nearly 80% tumor regression **(****FIGS. 7E-H)** at one-month follow-up. Median survival of 200 days nearly was three times greater than that (69 days) of comparable historic controls (Rossmeisl *et al.,* 2013).

In these examples, the inventors have identified the mechanistic underpinnings of a previously unrecognized and somewhat unexpected DC regulatory checkpoint, the full elucidation of which might be of critical importance to achieving clinical goals within the realm of vaccine immunotherapy. Previous and current work indicate that the simultaneous loading of DC with homologous class I and II antigens induces DC T_{H}1 polarization and an augmentation of downstream CD8⁺ T-cell responses *in vitro* and *in vivo* (Decker *et al.,* 2006; Decker *et al.,* 2009). Here the inventors demonstrate that this phenomenon is mechanistically-linked to upregulated secretion of AIMp1, an important cytokine with known T_{H}1 polarizing function, the release of which upregulates IL-12 secretion while concomitantly downregulating secretion of CTLA-4 and its corresponding mRNA transcript. AIMp1 appears to act upstream of both sCTLA-4 and IL-12 as demonstrated by AIMp1 siRNA knockdown and kinetic studies. Importantly, the inventors demonstrated that release of AIMp1 in response to homologous antigenic loading proceeded in a TLR-independent manner. TLR agonism was unable to augment AIMp1 release or diminish CTLA-4 secretion, nor were identically prepared peptide determinants able to stimulate such responses when added in a heterologous fashion. siRNA knockdown of β2-microglubulin or HLA-DM eliminated the ability of DC to respond to homologous class I and II binding peptides. Further, murine DC lacking H2-DM and thus unable to load exogenous antigen were also unable to be polarized or enhance downstream CD8⁺ responses though MHC was intact and pattern recognition receptors remained functional (Strong *et al.,* 2011). However, loading of H2-DM^{-/-} DC with a synthetic class I binding peptide that overlapped the amino acid sequence of Ii CLIP, theoretically the only possible manner by which to load class I and II of these cells in a homologous manner, released wild type ratios of AIMp1/sCTLA-4 and generated CD8⁺CD25⁺ T-cells in a dose responsive fashion. These results firmly suggest a role for peptide binding of MHC in this method of T_{H}1 polarization, a unique process not previously described. To further demonstrate T_{H}1 polarization dependent upon antigenic homology rather than innate PRR, the inventors added two non-anchor amino acid substitutions in a previously characterized (Decker *et al.,* 2009) class II binding peptide so that contiguous class I and II sequence homology of more than three amino acids was interrupted. This minor disruption of homology was sufficient to abrogate polarizing AIMp1/sCTLA-4 ratios. Applying the complimentary amino acid substitutions to the class I peptide, thereby restoring complete sequence homology, was sufficient to restore a high AIMp1/CTLA-4 ratio. Taken together, the data suggest a unique T_{H}1 polarizing checkpoint in DC dependent upon a high degree of sequence homology between MHC class I and II-bound peptides. In addition to identifying important effector molecules upon which this mechanism depends, the inventors demonstrated physiologic relevance through the generation of tissue-specific vaccines that broke immunological tolerance and eradicated normal immunologic self in the wild type mouse, a phenomenon not previously reported. Further experiments also demonstrated significant activity against neoplastic self in three different model systems, including experiments that suggested activity against oligodendroglioma in a spontaneous, outbred canine model.

In total, the inventors have used 29 different model systems **(Table 1),** including whole-cell systems, single-antigen systems, and multiple pairs of overlapping class I and II MHC binding epitopes to demonstrate that homologously-loaded DC exhibit a variety of T_{H}1-associated characteristics including differential cytokine secretion, surface marker expression, global transcriptional alterations, and the ability to enhance the generation of CD8⁺ CTL **(Table 2).** In each of these 29 different systems, the sole commonality among groups that displayed T_{H}1 polarization was the high degree of antigenic or amino acid sequence homology between the class I and class II antigens with which DC were loaded.

**Table 1. Summary of antigenic systems used, species tested, and method of antigen delivery to the DC. * = previously published system.**

| Antigen Source | Antigen Species | DC Species | Class I Determinant | Class II Determinant |
|---|---|---|---|---|
| Total Cellular Antigen Pools | | | | |
| Primary AML Sample 1* | Human | Human | mRNA | Lysate |
| Primary AML Sample 2* | Human | Human | mRNA | Lysate |
| Primary AML Sample 3* | Human | Human | mRNA | Lysate |
| TF-1a Erythroblast Cell Line* | Human | Human | mRNA | Lysate |
| FBMD-1 Stromal Cell Line* | Mouse | Human | mRNA | Lysate |
| PC-3 Prostate Cancer Cell Line | Human | Human | mRNA | Lysate |
| WPMY-1 Prostate Cell Line | Human | Human | mRNA | Lysate |
| RAW Macrophage Cell Line | Mouse | Human/Mouse | mRNA | Lysate |
| B16 Melanoma Cell Line | Mouse | Mouse | mRNA | Lysate |
| 4T-1 Breast Cancer Cell Line | Mouse | Mouse | mRNA | Lysate |
| TRAMP-C2 Prostate Cancer Cell Line | Mouse | Mouse | mRNA | Lysate |
| beta-TC-6 Pancreatic Beta Cell Line | Mouse | Mouse | mRNA | Lysate |
| Primary Seminal Vesicle | Mouse | Mouse | mRNA | Lysate |
| Primary Prostate | Mouse | Mouse | mRNA | Lysate |
| PROCAT/JOCK Prostatic Adenocarcinoma | Mouse | Mouse | mRNA | Lysate |
| Primary Oligodendrocytoma Sample 1 | Dog | Dog | mRNA | Lysate |
| Primary Oligodendrocytoma Sample 2 | Dog | Dog | mRNA | Lysate |
| Panc02 Pancreatic Cancer Cell Line | Mouse | Mouse | mRNA | Lysate |
| Kras^{G120}/p53^{-/-} PDAC Cell Line | Mouse | Mouse | mRNA | Lysate |
| THP-1 AML Cell Line | Human | Human | mRNA | Lysate |
| LN-18 Glioma Cell Line | Human | Human | mRNA | Lysate |
| Single Recombinant Antigen | | | | |
| GFP* | A. victoria | Human | Plasmid | rProtein |
| GFP | A. victoria | Human | Adenovirus | rProtein |
| GFP | A. victoria | Human/Mouse | mRNA | Protein |
| IL-4* | Mouse | Human | Plasmid | rProtein |
| HIV Nef | HIV | Human | Adenovirus | Peptide Library |
| TRP-2 | Mouse | Mouse | Adenovirus | Peptide |
| Ovalbumin | Chicken | Mouse | SIINFEKL | rProtein |
| Tc24 | T. Oruzi | Mouse | Adenovirus | rProtein |
| Overlapping MHC Binding Peptides | | | | |
| B8-166/DR3-162* | Influenza | Human | 8mer Peptide | 15mer Peptide |
| A3-172/DR3-162* | Influenza | Human | 9mer Peptide | 15mer Peptide |
| A2-443/DR3-440* | Influenza | Human | 9mer Peptide | 15mer Peptide |
| B8-166/DR3-162 Gly→Met | Synthetic | Human | 8mer Peptide | 15mer Peptide |
| CLIP | Mouse | Mouse H2-DM^{-/-} | 9mer Peptide | CLIP |

**Table 2. Select markers and manifestations of homologous DC loading.**

| **Surface Markers** | **Cytokines** | **Intracellular Signaling** | **Downstream Events** |
|---|---|---|---|
| **↑↑↑** CD83 | **↑↑↑** IL-12 secretion | **↑↑↑** Interferon signaling | **↑↑↑** Production of CD8⁺ T cells |
| **↑↑↑** C040 | **↑↑↑** AlMp1/p43 secretion | **↑↑↑** MyD88 | **↑↑↑** T coll IFNγ secretion |
| ↓↓↓ MHC dass II | ↓↓↓ CTLA-4 secretion | | **↑↑↑** CTL activity |
| | ↓↓↓ IL-23 secretion | | |
| | ↓↓↓ Wnt5a secretion | | |

### REFERENCES

U.S. Patent 5,788,963
U.S. Patent 8,728,806
Ahmed et al., J. Virol. 83, 2962-2975, 2009.
Allan et al., J. Immunol. 144, 3980-3986, 1990.
Ausubel, 1996.
Ausubel *et al.,* 1998.
Carstens et al., Cancer Res. 74, 609-620, 2014.
Carvalho et al., Infect Immun. 79, 1638-1646, 2011.
Decker et al., Vaccine 24, 3203-3216, 2006.
Decker et al., Blood 113, 4213-4223, 2009.
Dudda et al., Immunity 38, 742-753, 2013.
Ghosh et al., Nature 378, 457-462, 1995.
Hokey et al., Cancer Res. 65, 10059-10067, 2005.
Jones et al., Vaccine 17, 3065-3071, 1999.
Kim et al., Gene Ther. 15, 677-687, 2008.
Konduri et al., J. Infect Dis. 207, 1764-1772, 2013.
Longhi et al., J. Exp. Med. 206, 1589-1602, 2009.
Lopez et al., J. Infect Dis. 187, 1126-1136, 2003.
Lopez et al., J. Virol. 80, 3128-3134 A, 2006A.
Lopez et al., J. Virol. 80, 4538-4545, 2006B.
Lotze et al., eds. (2001). Dendritic Cells, Second Edition (San Diego: Academic Press).
Ressing et al., J. Immunother. 23, 255-266, 2000.
Rohn et al., Nat. Immunol. 5, 909-918, 2004.
Rossmeisl et al., J. Am. Vet. Med. Assoc. 242, 193-198, 2013.
Sambrook *et al.,* 1989.
Spranger et al., J. Immunol. 185, 738-747, 2010.
Tam and Wick, Immunology 128, 429-438. 2009.
Tripp et al., J. Immunol. 155, 2955-2959, 1995.

## Claims

1. A primed dendritic cell population for use in the treatment of a cancer or tumor in a subject, wherein the cells have been primed with at least one antigen associated with the cancer or at least one tumor antigen, and wherein the primed dendritic cell population is to be administered to the subject in conjunction with interferon-α (IFN-α).

2. The primed dendritic cell population for use according to claim 1, wherein the IFN-α is to be administered before the primed dendritic cell population.

3. The primed dendritic cell population for use according to claim 1, wherein the IFN-α is to be administered after the primed dendritic cell population.

4. The primed dendritic cell population for use according to claim 1, wherein the IFN-α is to be administered simultaneously with the primed dendritic cell population.

5. The primed dendritic cell population for use according to any one of claims 1 to 4, wherein (i) the IFN-α is to be administered systemically and the dendritic cell population is to be administered locally, or (ii) the IFN-α and the dendritic cell population are both to be administered locally.

6. The primed dendritic cell population for use according to any one of claims 1 to 5, wherein the primed dendritic cell population is to be administered to a lymphoid tissue site proximal to cancer cells or a tumor in the subject.

7. The primed dendritic cell population for use according to claim 6, wherein said lymphoid tissue site is a lymphoid tissue that drains tissue surrounding the cancer cells or the tumor.

8. The primed dendritic cell population for use according to any one of claims 1 to 7, wherein the primed dendritic cells have been homologously loaded with the antigen(s).

9. The primed dendritic cell population for use according to any one of claims 1 to 8, wherein further an immune checkpoint inhibitor is to be administered to the subject.

10. The primed dendritic cell population for use according to claim 9, wherein the immune checkpoint inhibitor is selected from the group consisting of ipilimumab, pembrolizumab, and nivolumab.

11. The primed dendritic cell population for use according to claim 9, wherein the immune checkpoint inhibitor is a CTLA-4 antagonist.

12. The primed dendritic cell population for use according to any one of claims 1 to 11, wherein the tumor is a solid tumor.

## Patentansprüche

1. Geprimte dendritische Zellpopulation zur Verwendung bei der Behandlung eines Krebs oder eines Tumors in einem Individuum, wobei die Zellen mit mindestens einem Antigen, das mit dem Krebs in Zusammenhang steht, oder mit mindestens einem Tumorantigen geprimt wurden, und wobei die geprimte dendritische Zellpopulation an das Individuum in Verbindung mit Interferon-α (IFN-α) zu verabreichen ist.

2. Geprimte dendritische Zellpopulation zur Verwendung nach Anspruch 1, wobei das IFN-α vor der geprimten dendritischen Zellpopulation zu verabreichen ist.

3. Geprimte dendritische Zellpopulation zur Verwendung nach Anspruch 1, wobei das IFN-α nach der geprimten dendritischen Zellpopulation zu verabreichen ist.

4. Geprimte dendritische Zellpopulation zur Verwendung nach Anspruch 1, wobei das IFN-α gleichzeitig mit der geprimten dendritischen Zellpopulation zu verabreichen ist.

5. Geprimte dendritische Zellpopulation zur Verwendung nach einem der Ansprüche 1 bis 4, wobei (i) das IFN-α systemisch zu verabreichen ist und die dendritische Zellpopulation lokal zu verabreichen ist, oder (ii) das IFN-α und die dendritische Zellpopulation beide lokal zu verabreichen sind.

6. Geprimte dendritische Zellpopulation zur Verwendung nach einem der Ansprüche 1 bis 5, wobei die geprimte dendritische Zellpopulation an eine Stelle des lymphatischen Gewebes in der Nähe von Krebszellen oder eines Tumors in dem Individuum zu verabreichen ist.

7. Geprimte dendritische Zellpopulation zur Verwendung nach Anspruch 6, wobei die Stelle des lymphatischen Gewebes ein lymphatisches Gewebe ist, das Gewebe drainiert, das die Krebszellen oder den Tumor umgibt.

8. Geprimte dendritische Zellpopulation zur Verwendung nach einem der Ansprüche 1 bis 7, wobei die geprimten dendritischen Zellen homolog mit dem Antigen/den Antigenen beladen wurden.

9. Geprimte dendritische Zellpopulation zur Verwendung nach einem der Ansprüche 1 bis 8, wobei des Weiteren ein Immuncheckpoint-Inhibitor an das Individuum zu verabreichen ist.

10. Geprimte dendritische Zellpopulation zur Verwendung nach Anspruch 9, wobei der Immuncheckpoint-Inhibitor ausgewählt ist aus der Gruppe bestehend aus Ipilimumab, Pembrolizumab und Nivolumab.

11. Geprimte dendritische Zellpopulation zur Verwendung nach Anspruch 9, wobei der Immuncheckpoint-Inhibitor ein CTLA-4-Antagonist ist.

12. Geprimte dendritische Zellpopulation zur Verwendung nach einem der Ansprüche 1 bis 11, wobei der Tumor ein solider Tumor ist.

## Revendications

1. Population de cellules dendritiques sensibilisées pour une utilisation dans le traitement d'un cancer ou d'une tumeur chez un sujet, dans laquelle les cellules ont été sensibilisées avec au moins un antigène associé au cancer ou au moins un antigène tumoral, et dans laquelle la population de cellules dendritiques sensibilisées doit être administrée au sujet en conjonction avec l'interféron-a (IFN-α).

2. Population de cellules dendritiques sensibilisées pour une utilisation selon la revendication 1, dans laquelle l'IFN-α doit être administré avant la population de cellules dendritiques sensibilisées.

3. Population de cellules dendritiques sensibilisées pour une utilisation selon la revendication 1, dans laquelle l'IFN-α doit être administré après la population de cellules dendritiques sensibilisées.

4. Population de cellules dendritiques sensibilisées pour une utilisation selon la revendication 1, dans laquelle l'IFN-α doit être administré simultanément avec la population de cellules dendritiques sensibilisées.

5. Population de cellules dendritiques sensibilisées pour une utilisation selon l'une quelconque des revendications 1 à 4, dans laquelle (i) l'IFN-α doit être administré par voie systémique et la population de cellules dendritiques doit être administrée localement, ou (ii) l'IFN-α et la population de cellules dendritiques doivent tous deux être administrés localement.

6. Population de cellules dendritiques sensibilisées pour une utilisation selon l'une quelconque des revendications 1 à 5, dans laquelle la population de cellules dendritiques sensibilisées doit être administrée à un site de tissu lymphoïde proche de cellules cancéreuses ou d'une tumeur chez le sujet.

7. Population de cellules dendritiques sensibilisées pour une utilisation selon la revendication 6, dans laquelle ledit site de tissu lymphoïde est un tissu lymphoïde qui draine le tissu entourant les cellules cancéreuses ou la tumeur.

8. Population de cellules dendritiques sensibilisées pour une utilisation selon l'une quelconque des revendications 1 à 7, dans laquelle les cellules dendritiques sensibilisées ont été chargées de manière homologue avec le ou les antigènes.

9. Population de cellules dendritiques sensibilisées pour une utilisation selon l'une quelconque des revendications 1 à 8, dans laquelle un inhibiteur de point de contrôle immunitaire doit en outre être administré au sujet.

10. Population de cellules dendritiques sensibilisées pour une utilisation selon la revendication 9, dans laquelle l'inhibiteur de point de contrôle immunitaire est choisi dans le groupe constitué par l'ipilimumab, le pembrolizumab et le nivolumab.

11. Population de cellules dendritiques sensibilisées pour une utilisation selon la revendication 9, dans laquelle l'inhibiteur de point de contrôle immunitaire est un antagoniste de CTLA-4.

12. Population de cellules dendritiques sensibilisées pour une utilisation selon l'une quelconque des revendications 1 à 11, dans laquelle la tumeur est une tumeur solide.
